# EUROPÄISCHE PATENTSCHRIFT

(11) **EP 2 885 018 B1**
(45) Veröffentlichungstag und Bekanntmachung des Hinweises auf die Patenterteilung: **01.05.2019**
(21) Anmeldenummer: 13700102.0
(22) Anmeldetag: 09.01.2013
(51) Int. Cl.: A61L 31/18, A61M 25/01, A61M 25/09

(54) **MR- ODER HF-FÄHIGER MEDIZINISCHER FÜHRUNGSDRAHT**
MR-CAPABLE OR HF-CAPABLE MEDICAL GUIDE WIRE
FIL DE GUIDAGE MÉDICAL COMPATIBLE AVEC L'IMAGERIE PAR RÉSONANCE MAGNÉTIQUE OU À HAUTE FRÉQUENCE

(30) Priorität: 20.08.2012 DE 102012214785
(43) Veröffentlichungstag der Anmeldung: 24.06.2015
(73) Patentinhaber: Epflex Feinwerktechnik GmbH, 72581 Dettingen/Erms (DE)
(72) Erfinder: UIHLEIN, Bernhard, 72581 Dettingen (DE)
(74) Vertreter: Patentanwälte Ruff, Wilhelm, Beier, Dauster & Partner mbB
(86) Internationale Anmeldenummer: PCT/EP2013/050331
(87) Internationale Veröffentlichungsnummer: WO 2014/029508

(56) Entgegenhaltungen:
- WO-A1-98/55016
- WO-A2-03/092791
- DE-A1-102005 022 688

## Beschreibung

Die Erfindung bezieht sich auf einen medizinischen Führungsdraht mit einem Drahtkern aus MR-unsichtbarem Material, einer den Drahtkern wenigstens abschnittweise und mit Berührkontakt umgebenden Ummantelung und einem MR-Marker aus MR-sichtbarem Material. Die Erfindung bezieht sich zudem auf einen für HF(Hochfrequenz)-Anwendungen geeigneten medizinischen Führungsdraht.

Unter MR-Marker wird vorliegend eine Komponente des Führungsdrahtes verstanden, die eine Sichtbarmachung desselben in Magnetresonanztomographie-Anwendungen, abgekürzt MRT- oder auch MR-Anwendungen, einschließlich Kernmagnetresonanz(NMR)-Anwendungen ermöglicht. Mit der Charakterisierung "MR-sichtbar" sind dementsprechend Materialien gemeint, die in derartigen MR-Anwendungen detektierbare Artefakte zeigen, d.h. sichtbar sind, im Gegensatz zu "MR-unsichtbaren" Materialien, die keine solchen Artefakte zeigen und daher in derartigen Anwendungen nicht sichtbar sind. Der Führungsdraht ist im Übrigen selbstverständlich so ausgeführt, dass er für derartige Anwendungen tauglich ist. Dazu gehört meist die Wahl eines nichtmetallischen Materials für den Drahtkern, typischerweise eines Kunststoffmaterials.

Es sind bereits verschiedentlich MR-sichtbare Führungsdrähte vorgeschlagen geworden. So ist in der Offenlegungsschrift WO 2007/000148 A2 ein Führungsdraht offenbart, der aus einem oder mehreren Stäben und einem den oder die Stäbe umschließenden und/oder miteinander verklebenden, nicht-ferro-magnetischen Matrixwerkstoff aufgebaut ist. Der jeweilige Stab besteht aus einem oder mehreren nichtmetallischen Filamenten und einem selbige umschließenden und/oder miteinander verklebenden, nicht-ferro-magnetischen Matrixwerkstoff, der mit einem MR-Marker dotiert ist. Als MR-Marker werden geeignete Nanopartikel vorgeschlagen, als Matrixwerkstoff ein Epoxidharz.

In der Offenlegungsschrift WO 2009/141165 A2 wird in ähnlicher Weise ein Führungsdraht vorgeschlagen, der zumindest einen Stab aus schlecht elektrisch leitendem Material aufweist, welcher aus einem Matrixwerkstoff und nichtmetallischen Filamenten, wie Glasfasern, Keramikfasern, pflanzliche Fasern oder Kunststofffasern, aufgebaut ist. Der Oberflächenbereich des so gebildeten Instrumentenkörpers ist mit einem immobilisierten aktiven MR-Marker aus bestimmten chemischen Substanzen versehen, oder es ist ein passiver MR-Marker in den Matrixwerkstoff des stabförmigen Instrumentenkörpers dotiert, z.B. in Form spezieller Metallpartikel, die gleichzeitig als Röntgen-Marker fungieren können.

Die Offenlegungsschrift WO 01/95794 A1 offenbart einen Führungsdraht, der durch eine Röhre aus Polymermaterial gebildet ist, in deren hohlen Innenraum ein MR-Marker eingebracht ist, wobei er gleichzeitig als Röntgen-Marker fungieren kann oder zusätzlich ein Röntgen-Marker vorgesehen sein kann. Als MR-Markermaterial werden insbesondere Salze und Oxide von Dysprosium vorgeschlagen. In einer Ausführungsvariante erstreckt sich ein dünner Glasfaserfaden unter Belassung eines radialen Freiraums durch das hohle Innere der Polymerröhre hindurch und dient als Halterung für mehrere axial beabstandete MR-Markerelemente.

In der Offenlegungsschrift DE 10 2008 006 402 A1 wird zur MR-Sichtbarkeit von Instrumenten für die Invasivmedizin eine Beschichtung mit einem Ferrofluid vorgeschlagen, das paramagnetische Eisenoxid-Nanopartikel enthält.

Die Patentschrift DE 10 2006 020 402 B3 offenbart einen Führungsdraht für einen auf Gehirnuntersuchungen ausgelegten Katheter, wobei der Führungsdraht magnetische Nanopartikel in Flüssigkeit suspendiert und/oder in Pulverform umfasst, um ihn durch Anlegen äußerer Magnetfelder zu einem Ziel bewegen zu können.

Viele herkömmliche medizinische Führungsdrähte bestehen aus einem Drahtkern und einer einlagigen Ummantelung, wobei der Drahtkern aus einem Material mit höherer Biegesteifigkeit als die Ummantelung gebildet ist, so dass er die Biegesteifigkeit des Führungsdrahtes insgesamt bestimmt. Aus diesem Grund ist der Drahtkern häufig zum vorderen, distalen Ende hin verjüngt, um in diesem Einsatzbereich die Biegesteifigkeit des Führungsdrahtes zu reduzieren. Oftmals ist die Ummantelung noch zusätzlich mit einer an den Anwendungsfall angepassten, z.B. hydrophilen Oberflächenbeschichtung versehen.

In der Patentschrift DE 10 2005 022 688 B4 ist ein Führungsdraht offenbart, bei dem der Drahtkern nur in einem an einen distalen Abschnitt anschließenden Schaftabschnitt von einer gegenüber dem Drahtkern biegesteiferen Ummantelung umgeben ist. Bei diesem Typ von Führungsdraht ist folglich die Biegesteifigkeit des Schaftabschnitts durch die Ummantelung, die z.B. aus einem PEEK(Polyetheretherketon)-Material oder einem Polyimid-Material besteht, und nicht durch den Drahtkern bestimmt. Der biegeweichere Drahtkern braucht daher im distalen Abschnitt nicht zwingend verjüngt werden, um eine für den distalen Abschnitt gegenüber dem Schaftabschnitt gewünschte geringere Biegesteifigkeit bereitzustellen. Des Weiteren sind bei diesem Führungsdraht MR-Marker in Form von Füllkugeln oder Hohlräumen, die mit geeigneten Fremdstoffen dotiert sein können, in die distale Umhüllung des Drahtkerns eingebracht.

Die Offenlegungsschrift WO 03/092791 A2 offenbart einen Führungsdraht mit einer mehrlagigen Beschichtung, die eine erste Schichtlage, welche ein biologisch aktives Material enthält, und eine darauf aufgebrachte, zweite Schichtlage beinhaltet, die ein Polymermaterial und magnetische Partikel enthält und frei von dem biologisch aktiven Material ist. Das biologisch aktive Material soll im Körper eines Patienten freigesetzt werden, vorzugsweise unter Einsatz einer elektromagnetischen Energiequelle oder einer mechanischen Vibrationsenergiequelle. Die magnetischen Partikel können außerdem eine MR-Markerfunktion erfüllen.

Die Offenlegungsschrift WO 98/55016 A1 offenbart einen Führungsdraht mit einem Drahtkern aus Glas und einer mehrlagigen Beschichtung, die eine Schutzbeschichtung aus Polyimid, eine Kleberschicht und eine Polymerhülle umfasst, wobei in einem aus Kunststoff bestehenden distalen Endabschnitt ein MR-Marker angeordnet ist. Eine Mehrzahl von Verstärkungsfasern ist zur Verbesserung der Flexibilitäts- und Verwindungseigenschaften des Führungsdrahtes am Drahtkern und/oder an der Polymerhülle angebracht.

Der Erfindung liegt als technisches Problem die Bereitstellung eines medizinischen Führungsdrahtes der eingangs genannten Art zugrunde, der hinsichtlich Biegesteifigkeitsverhalten und/oder MR-Sichtbarkeit und/oder Verwendbarkeit in HF-Anwendungen gegenüber dem obengenannten Stand der Technik weiter verbessert ist und sich mit relativ geringem Aufwand und hoher Funktionszuverlässigkeit fertigen lässt.

Die Erfindung löst dieses Problem durch die Bereitstellung eines Führungsdrahtes mit den Merkmalen des Anspruchs 1 oder 4. Beim Führungsdraht gemäß Anspruch 1 besitzt die Ummantelung einen mehrlagigen Schichtaufbau, der zwei oder mehr übereinanderliegende Vollmaterial-Schichtlagen und wenigstens eine Faser-Schichtlage aus unterschiedlichen, MR-unsichtbaren Kunststoffmaterialien beinhaltet. Nicht mitgezählt ist hierbei eine optionale, typischerweise sehr dünne, z.B. hydrophile Oberflächenbeschichtung üblicher Art, mit der die Ummantelung an ihrer Außenseite versehen sein kann. Der MR-Marker weist wenigstens ein MR-Markerelement auf, das wenigstens teilweise in die Ummantelung integriert oder von dieser umgeben ist. Beim Führungsdraht gemäß Anspruch 4 ist der Drahtkern wenigstens abschnittsweise und mit Berührkontakt von einer elektrisch isolierenden Ummantelung umgeben, die einen mehrlagigen Schichtaufbau mit zwei oder mehr übereinanderliegenden Vollmaterial-Schichtlagen und wenigstens eine dazwischen angeordnete Faser-Schichtlage aus unterschiedlichen Kunststoffmaterialien beinhaltet. Diese elektrisch isolierende Auslegung macht den Führungsdraht sehr gut für HF-Anwendungen geeignet, wobei der Drahtkern bei Bedarf auch aus einem metallischen Material bestehen kann, wie aus einer superelastischen Nickel-Titan-Legierung.

Spezieller weist der Ummantelungs-Schichtaufbau wenigstens zwei Vollmaterial-Schichtlagen und wenigstens eine dazwischen angeordnete Faser-Schichtlage auf, die durch ein Gewebe- oder Fasermaterial gebildet ist, wobei die Faser-Schichtlage durch ein Gewebe- oder Fasermaterial aus einer einlagigen, lückenlosen Anordnung von Fasern gebildet ist, die parallel nebeneinanderliegend oder mit einer wählbaren axialen Fasersteigung schräg verlaufend oder schraubenförmig um die darunterliegende Vollmaterial-Schichtlage gewickelt angeordnet sind. Dies kann weiter zur Erzielung einer gewünschten hohen Biegesteifigkeit insbesondere bei vergleichsweise dünnen Führungsdrähten beitragen.

Durch den spezifischen mehrlagigen Aufbau lässt sich die Ummantelung des Drahtkerns optimal auf die Erfordernisse des Anwendungsfalls abstimmen. Insbesondere zeigt sich, dass sich, wenn gewünscht, die Ummantelung bei gegebener Führungsdrahtdicke durch den Mehrlagenaufbau auf Wunsch mit gegenüber dem Drahtkern deutlich höherer Biegesteifigkeit unter Beibehaltung der sonstigen, für Führungsdrähte geforderten Eigenschaften realisieren lässt, so dass die Biegesteifigkeit des Führungsdrahtes in dem Abschnitt, in welchem die Ummantelung vorhanden ist, praktisch ausschließlich durch die Ummantelung und nicht durch den Drahtkern bestimmt ist. Dazu kann beispielsweise die Wahl eines sehr harten, spröden Materials für eine der Vollmaterial-Schichtlagen und eines sehr zähen, festen Materials für eine andere der Vollmaterial-Schichtlagen beitragen. Der Drahtkern braucht folglich nicht auf die Erzielung einer entsprechend hohen Biegesteifigkeit im ummantelten Abschnitt ausgelegt werden, sondern kann hinsichtlich anderer Charakteristika optimiert werden. Das wenigstens teilweise in die mehrlagige Ummantelung integrierte oder von dieser umgebene MR-Markerelement gewährleistet eine gewünschte MR-Sichtbarkeit des Führungsdrahtes im entsprechenden Bereich.

In einer vorteilhaften Weiterbildung der Erfindung gemäß Anspruch 4 weist der für HF-Anwendungen geeignete Führungsdraht zusätzlich die Merkmale des MR-fähigen Führungsdrahtes nach Anspruch 1 auf und eignet sich auf diese Weise sowohl für HF- als auch für MR-Anwendungen.

In einer Weiterbildung der Erfindung trägt zu einer hohen Biegesteifigkeit bei, dass die Ummantelung mindestens zwei Faser-Schichtlagen mit unterschiedlichen axialen Fasersteigungen und/oder Faserwickelrichtungen aufweist.

Der Drahtkern ist in einer Weiterbildung der Erfindung als Drahtlitze aus mehreren seil- oder litzenbildend verbundenen Einzeldrähten gebildet. Bei den Einzeldrähten handelt es sich beispielsweise um monofile Kunststofffäden oder um Einzeldrahtlitzen aus Kunststoff. In weiterer Ausgestaltung weist der MR-Marker ein MR-sichtbares Material auf, das in Zwischenräume zwischen den Einzeldrähten der so gebildeten Drahtkernlitze eingebracht ist. Dies realisiert einen kernnahen MR-sichtbaren Bereich des Führungsdrahtes, ohne dass dazu der Drahtkern selbst MR-sichtbar gefertigt werden braucht.

In einer vorteilhaften Weiterbildung der Erfindung umgibt die Ummantelung den Drahtkern wenigstens in einem an einen distalen Abschnitt anschließenden Schaftabschnitt, jedoch nicht im distalen Abschnitt, und ist mit einer höheren Biegesteifigkeit ausgeführt als der Drahtkern. Im Schaftabschnitt bestimmt daher die Ummantelung die Biegesteifigkeit des Führungsdrahtes. Im distalen Abschnitt kann der biegeweichere Drahtkern die Biegesteifigkeit bestimmen, so dass der distale Abschnitt insgesamt biegeweicher bleibt als der Schaftabschnitt, wie dies für viele Führungsdrahtanwendungen gewünscht ist. Günstig ist hierbei, dass der Drahtkern zu diesem Zweck im distalen Abschnitt nicht zwingend verjüngt werden braucht, was entsprechenden Fertigungsaufwand einspart.

In Ausgestaltung der Erfindung sind die Vollmaterial-Schichtlagen der Ummantelung aus biegesteifen Kunststoffmaterialien gefertigt, wie ABS(Acrylnitril-Butadien-Styrol)-, PEEK-, PET(Polyethylenterephthalat)-, Ultramid- und/oder Epoxidharz-Materialien. Diese Realisierung eignet sich insbesondere für Führungsdrähte, die im Bereich der Ummantelung eine relativ hohe Biegesteifigkeit haben sollen.

In einer Ausgestaltung der Erfindung sind die Faser-Schichtlagen aus Glasfasern, Aramid- bzw. Kevlar-Fasern, und/oder Polyesterfasern gebildet. Dazu kann eine einzelne Faser oder ein Satz mehrerer paralleler Fasern auf den jeweiligen Untergrund gewickelt bzw. aufgebracht sein, oder die Fasern können zu einem Gewebe geflochten sein, das zur Bildung der betreffenden Schichtlage aufgelegt ist.

In einer Weiterbildung der Erfindung beinhaltet der MR-Marker ein MR-Markerelement, das in eine der Ummantelungs-Schichtlagen oder zwischen zwei der Ummantelungs-Schichtlagen oder zwischen den Drahtkern und die angrenzende Ummantelungs-Schichtlage eingebettet ist. Dies stellt herstellungstechnisch günstige Möglichkeiten zur Bereitstellung einer MR-Sichtbarkeit des Führungsdrahtes im Bereich der Ummantelung dar.

In weiterer Ausgestaltung bieten sich für die Realisierung eines solchen MR-Markerelementes mehrere, sich nicht gegenseitig ausschließende Alternativen an. So können MR-sichtbare Partikel in eine oder mehrere der Vollmaterial-Schichtlagen eingebettet sein. Oder es können ein oder mehrere MR-Linienelemente in die Ummantelung eingebettet sein, z.B. ein über die axiale Länge der Ummantelung geradlinig, schraubenförmig oder mit einem anderen Verlauf durchgehendes Linienelement oder ein Satz mehrerer, gegenüber der Ummantelung axial kürzerer Linienelemente, die mit oder ohne Versatz in Umfangsrichtung und mit oder ohne axialem Versatz sowie mit gleicher oder unterschiedlicher Länge angeordnet sind. Des weiteren kann ein solches MR-Markerelement durch eine der Fasern einer betreffenden Faser-Schichtlage gebildet sein, wozu die Faser entsprechend ein MR-sichtbares Material enthält, z.B. durch Dotierung von MR-sichtbaren Partikeln in das Fasermaterial oder durch Herstellen der Faser aus einem MR-sichtbaren Material oder durch Beschichten der Faser mit einem MR-sichtbaren Material. Bei Bedarf kann das MR-Markerelement auch ein oder mehrere MR-Linienelemente beinhalten, die als Längenmessmarkerelemente ausgeführt sind und dadurch eine Längenmessanwendung bei dem Führungsdraht unterstützen.

In Weiterbildung der Erfindung ist ein MR-sichtbares, draht- oder rohrförmiges Hilfselement aus nichtmagnetischem Material in einem distalen Führungsdrahtabschnitt angeordnet. Mit diesem Hilfselement lässt sich die MR-Sichtbarkeit dieses distalen Führungsdrahtabschnitts gezielt verstärken. Zudem lassen sich je nach Ausführung des Hilfselements die elastischen Eigenschaften dieses distalen Führungsdraht-Endabschnitts in gezielter Weise beeinflussen bzw. verbessern, beispielsweise zur Erzielung bestimmter formgebender Eigenschaften, wie die Erzielung eines abgewinkelten distalen Endbereichs oder eines J-förmigen distalen Spitzenabschlusses des Führungsdrahtes. Dazu kann das Hilfselement z.B. aus einem nichtmagnetischen Metallmaterial bestehen.

In vorteilhafter Ausgestaltung kann das Hilfselement einen Hilfselementdraht, der den Drahtkern schraubenlinienförmig umgibt und/oder sich mit axialer Hauptkomponente neben und entlang des Drahtkerns erstreckt, und/oder ein Hilfselementrohr umfassen, das den Drahtkern in einem entsprechenden axialen Abschnitt umgibt.

In weiterer Ausgestaltung dieses Erfindungsaspektes wird die MR-Sichtbarkeit des Hilfselementes dadurch bereitgestellt oder erhöht, dass es mit einem MR-Markermaterial dotiert und/oder beschichtet ist.

Vorteilhafte Ausführungsformen der Erfindung sind in den Zeichnungen dargestellt und werden nachfolgend beschrieben. Hierbei zeigen:
- Fig. 1: eine verkürzte Längsschnittansicht eines Führungsdrahtes mit dreilagiger schaftseitiger Ummantelung,
- Fig. 2: eine Querschnittansicht längs einer Linie II-II von Fig. 1,
- Fig. 3: eine teilgeschnittene Perspektivansicht des Führungsdrahtes von Fig. 1,
- Fig. 4: eine verkürzte Längsschnittansicht eines Führungsdrahtes mit fünflagiger Ummantelung,
- Fig. 5: eine Querschnittansicht längs einer Linie V-V von Fig. 4,
- Fig. 6 bis 10: teilgeschnittene Perspektivansichten von Führungsdrahtvarianten mit achtlagiger Ummantelung und unterschiedlich eingebrachten MR-Markerelementen,
- Fig. 11: eine Perspektivansicht eines MR-Markerelementgerüstes bei einer weiteren Führungsdrahtvariante,
- Fig. 12: eine verkürzte Längsschnittansicht einer Führungsdrahtvariante mit einem distalen Hilfselementdraht,
- Fig. 13: eine Ansicht entsprechend Fig. 12 für eine Hilfsdrahtvariante mit Pilzkopfabschlüssen,
- Fig. 14: eine Ansicht entsprechend Fig. 12 für eine Hilfsdrahtvariante mit distalem Ringabschluss,
- Fig. 15: eine Ansicht entsprechend Fig. 12 für eine Hilfsdrahtvariante mit proximalem Ringabschluss,
- Fig. 16: eine Ansicht entsprechend Fig. 12 mit zusätzlichem Ringelement für den Hilfselementdraht,
- Fig. 17: eine Ansicht entsprechend Fig. 12 für eine Variante mit schraubenlinienförmigem Hilfselementdraht,
- Fig. 18: eine Ansicht entsprechend Fig. 17 für eine Variante mit zusätzlichem axialem Hilfselementdrahtstück,
- Fig. 19: eine Seitenansicht eines in den Varianten der Fig. 12 bis 18 verwendbaren Hilfselementdrahtes,
- Fig. 20: eine Ansicht entsprechend Fig. 19 für eine weitere Hilfsdrahtvariante,
- Fig. 21: eine Ansicht entsprechend Fig. 12 für eine Führungsdrahtvariante mit distaler Hilfselementhülse über dem Drahtkern,
- Fig. 22: eine Ansicht entsprechend Fig. 21 mit einer weiteren Hilfselementhülsenvariante,
- Fig. 23: eine Ansicht entsprechend Fig. 21 mit noch einer weiteren Hilfselementhülsenvariante,
- Fig. 24 bis 29: je eine Seitenansicht verschiedener verwendbarer Hilfselementhülsen zum distalen Umgeben des Drahtkerns,
- Fig. 30: eine Längsschnittansicht einer Hilfselementhülse mit MR-sichtbar dotiertem Hülsenmaterial,
- Fig. 31: eine Querschnittansicht längs einer Linie XXXI-XXXI von Fig. 30,
- Fig. 32: eine Querschnittansicht entsprechend Fig. 31 für eine Hilfselementhülsenvariante mit MR-sichtbarer Oberflächendotierung,
- Fig. 33: eine Seitenansicht einer Hilfselementhülsenvariante mit partieller MR-Oberflächendotierung,
- Fig. 34: eine Seitenansicht entsprechend Fig. 33 für eine Hilfselementhülsenvariante mit zwei unterschiedlichen MR-Oberflächendotierungen,
- Fig. 35: eine Seitenansicht einer Hilfselementhülsenvariante aus einem Materialgeflecht und
- Fig. 36 bis 38: je eine Seitenansicht weiterer Hilfselementhülsenvarianten mit unterschiedlichen Einschnitten.

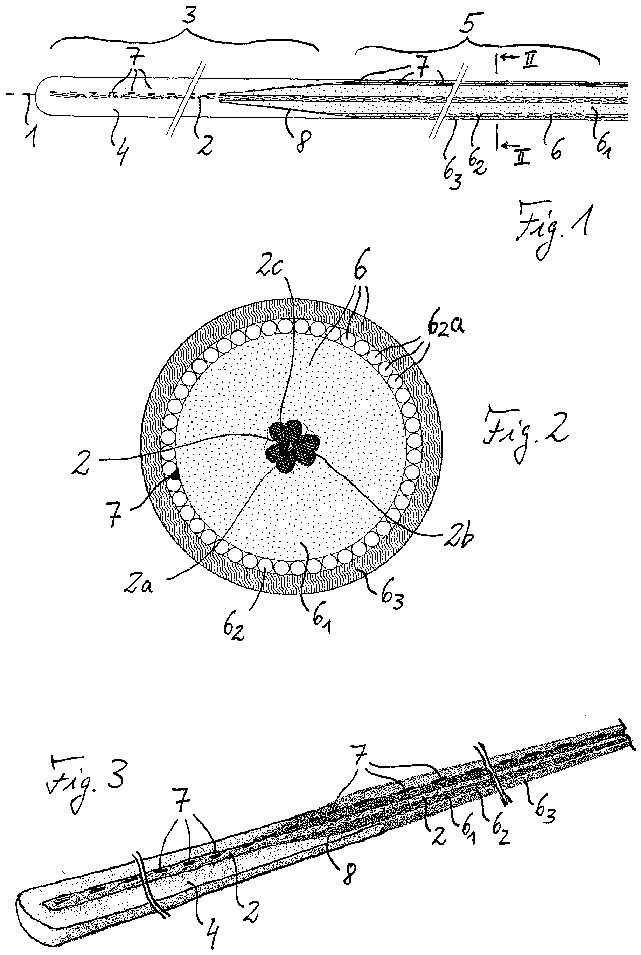

Der in den Fig. 1 bis 3 gezeigte Führungsdraht weist als zentrisches Element entlang seiner Längsmittenachse 1 einen Drahtkern 2 auf, der in einem vorderen, distalen Führungsdrahtabschnitt 3 von einer biegeweicheren Umhüllung 4 und in einem daran anschließenden Schaftabschnitt 5 von einer biegesteiferen Ummantelung 6 umgeben ist. Insbesondere besitzt die Ummantelung 6 eine höhere Biegesteifigkeit als der Drahtkern 2, so dass die Biegesteifigkeit des Führungsdrahtes im Schaftbereich 5 durch diejenige der Ummantelung 6 bestimmt ist. Hingegen besteht die distale Umhüllung 4 aus einem gegenüber der Schaftummantelung 6 deutlich biegeweicheren Material, so dass die Flexibilität des distalen Führungsdrahtabschnitts 3 höher als im Bereich der Schaftummantelung 6 ist. Da die Biegesteifigkeit des Führungsdrahtes im Schaftbereich 5 durch die Ummantelung 6 bestimmt ist, kann der Drahtkern 2 insgesamt auf die Erzielung einer gewünschten Biegesteifigkeit des distalen Abschnitts 3 ausgelegt werden, ohne dass er dazu im distalen Abschnitt 3 zwingend anders gestaltet sein muss als im Schaftabschnitt 5. So besitzt der Drahtkern 2 im gezeigten Beispiel im gesamten distalen Abschnitt 3 den gleichen Durchmesser wie im Schaftabschnitt 5. Es entfällt daher ggf. die Notwendigkeit, den Drahtkern 2 im distalen Abschnitt 3 abschleifen oder in anderer Weise mit einem geringeren Durchmesser als im Schaftabschnitt 5 herstellen zu müssen, um für den distalen Abschnitt 3 eine geringere Biegesteifigkeit als für den Schaftabschnitt 5 zu erzielen.

Der Drahtkern 2 ist im gezeigten Beispiel durch ein Litzenmaterial aus drei Einzeldrähten 2a, 2b, 2c gebildet, die ihrerseits jeweils wieder als Drahtseile oder Drahtlitzen gefertigt sind, und verläuft einteilig vom distalen Ende bis zu einem proximalen, hinteren Führungsdrahtabschluss 9. Alternativ können die Einzeldrähte auch als monophile Drahtstücke realisiert sein. Ebenso kann in alternativen Ausführungsformen der Drahtkern 2 insgesamt aus einem monophilen Drahtstück oder einem komplexen Drahtgeflecht bestehen. In jedem Fall besteht der Drahtkern 2 aus einem MR-unsichtbaren Material, vorzugsweise aus einem relativ elastischen und zähen, hochfesten Kunststoffmaterial, wobei jegliches derartige Material verwendbar ist, das dem Fachmann für diesen Anwendungszweck an sich bekannt ist.

Die Ummantelung 6 weist einen mehrlagigen Schichtaufbau auf, im gezeigten Beispiel speziell einen dreilagigen Schichtaufbau mit einer inneren Vollmaterial-Schichtlage 6₁, einer äußeren Vollmaterial-Schichtlage 6₃ und einer zwischenliegenden Faser-Schichtlage 6₂. Die beiden Vollmaterial-Schichtlagen 6₁, 6₃ bestehen vorzugsweise aus unterschiedlichen Materialien bzw. Materialkomponenten. Als Materialien hierfür eignen sich insbesondere biegesteife Werkstoffe wie ABS-, PET-, Ultramid- und/oder Epoxidharz-Kunststoffe, die optional mit Füllstoffen versehen sein können, wobei auch hierfür wiederum grundsätzlich alle Materialien zur Verwendung in Betracht kommen können, die dem Fachmann für diesen Anwendungszweck an sich bekannt sind. So kann z.B. für die eine der beiden Vollmaterial-Schichtlagen 6₁, 6₃ ein relativ zäher, fester Werkstoff und für die andere ein relativ harter, spröder Werkstoff verwendet werden. In jedem Fall handelt es sich bei den Materialien für die Vollmaterial-Schichtlagen 6₁, 6₃ der Ummantelung 6 ebenso wie beim Material für den Drahtkern 2 um MR-unsichtbare Materialien.

Es sei an dieser Stelle erwähnt, dass beim erfindungsgemäßen Führungsdraht die aufeinanderfolgenden Schichtlagen der Ummantelung in radialer Richtung mit Berührkontakt eng aneinander anliegen und die innerste Schichtlage mit Berührkontakt eng am Drahtkern anliegt, wie aus Fig. 2 für das dortige Beispiel mit dem Drahtkern 2 und den drei Ummantelungs-Schichtlagen 6₁, 6₂, 6₃ deutlich zu erkennen. Optional kann die äußerste Ummantelungs-Schichtlage, in Fig. 2 die Schichtlage 6₃, mit einer Oberflächenbeschichtung z.B. hydrophiler Art versehen sein, wie sie dem Fachmann an sich bekannt ist. Eine solche Oberflächenbeschichtung wird hierbei vorliegend nicht als eine Schichtlage der Ummantelung 6, sondern als optionale, zusätzliche Außenbeschichtung angesehen.

Um den Führungsdraht zum Einsatz in MR-Anwendungen geeignet zu machen, ist er mit einem MR-Marker versehen. Im gezeigten Beispiel der Fig. 1 bis 3 besteht dieser MR-Marker aus einer Mehrzahl von streifenförmigen MR-Markerelementen 7, die über die gesamte Führungsdrahtlänge vorgesehen sind. Im distalen Bereich 3 befinden sie sich in Zwischenräumen des Drahtlitzenkerns 2 bzw. sind angrenzend an den Drahtkern 2 in die Umhüllung 4 eingebettet. Im Schaftabschnitt 5 sind sie in die Ummantelung 6 integriert, im gezeigten Beispiel speziell in die Faser-Schichtlage 6₂. Im Beispiel der Fig. 1 bis 3 sind die MR-Markerelemente 7 mit axialem Abstand voneinander längs einer Linie angeordnet, alternativ sind auch beliebige andere Anordnungen möglich, beispielsweise solche mit gegenseitigem Versatz in Führungsdraht-Umfangsrichtung. Für die MR-Markerstreifen 7 kann wiederum jegliches herkömmliche, MR-sichtbare Material verwendet werden, das dem Fachmann für diesen Anwendungszweck an sich bekannt ist.

Vorteilhaft können die MR-Markerelemente 7 diskriminierbar unterschiedlich im von der Ummantelung 6 umgebenen Schaftabschnitt 5 einerseits und im von der Ummantelung 6 freien, distalen Bereich 3 andererseits gestaltet sein, beispielsweise mit kürzerer Länge und kürzerem axialem Abstand im distalen Bereich 3 und größerer Länge und größerem Abstand im Schaftabschnitt 5. Im gezeigten Beispiel verläuft die Anordnungslinie der MR-Markerelemente 7 in einer Längsebene des Führungsdrahtes. Alternativ kann diese Anordnungslinie auch schraubenförmig gewunden verlaufen. In entsprechenden Ausführungsformen sind die MR-Markerelemente 7 als Längenmessmarker ausgeführt, die eine Längenmessung am Führungsdraht über die Detektion der MR-Markerelemente 7 gestatten. Dazu können die Abstände zwischen den aufeinanderfolgenden MR-Markerelementen 7 und/oder ihre axiale Ausdehnung jeweils eine vorgegebene, definierte Länge aufweisen. Diese definierte Länge der MR-Markerelemente 7 bzw. ihrer Abstände längs der Anordnungslinie kann hierbei, wenn gewünscht, im distalen Bereich 3 anders gewählt sein als im Schaftabschnitt 5. In entsprechenden Ausführungsformen der Erfindung sind zusätzlich zu den längs einer Linie angeordneten MR-Markerelementen 7 weitere derartige, im Wesentlichen axial verlaufende MR-Markerstreifen vorgesehen, die zur Bildung entsprechender Markerringe in Umfangsrichtung gegeneinander versetzt und axial z.B. auf Höhe jeweils eines der entlang der Anordnungslinie befindlichen MR-Markerelemente 7 angeordnet sind, wobei die Markerringe ihrerseits vorzugsweise in einem regelmäßigen axialen Abstand voneinander positioniert sind. So kann beispielsweise jedes n-te MR-Markerelement 7, das sich auf der Anordnungslinie befindet, durch zusätzliche, gleichartige, in Umfangsrichtung zu diesem versetzt angeordnete Markerstreifen zu einem derartigen Markerring ergänzt sein, mit n als einer beliebig wählbaren ganzen Zahl größer als eins. Dies unterstützt die Verwendung eines solchen MR-Markers zur erwähnten Längenmessung.

Für die Faser-Schichtlage 6₂ sind beispielsweise Glasfasern, Aramid- bzw. Kevlarfasern oder Polyesterfasern geeignet. Im gezeigten Beispiel besteht die Faser-Schichtlage 6₂ aus einer einlagigen, lückenlosen Anordnung von parallel nebeneinanderliegenden Fasern 6₂a, die in axialer Richtung verlaufend angeordnet sind. Alternativ können die Fasern 6₂a um die darunterliegende Vollmaterial-Schichtlage 6₁ mit einer wählbaren Wickelrichtung und einer wählbaren axialen Fasersteigung schräg verlaufend bzw. schraubenförmig gewickelt sein.

Im gezeigten Beispiel schließt die distale Umhüllung 4 in axialer Richtung nicht abrupt an die Schaftummantelung 6 an, sondern unter Bildung eines stetigen, konischen Übergangs 8. Herstellungstechnisch lässt sich diese spezielle Ausführungsform des Führungsdrahtes beispielsweise mit relativ geringem Aufwand dadurch realisieren, dass der Drahtkern 2 zunächst auf seiner ganzen Länge mit der mehrlagigen Ummantelung 6 versehen wird, anschließend im distalen Abschnitt 3 unter Bildung einer konischen Verjüngung im Übergangsbereich 8 abgetragen und der distal freigelegte Drahtkern 2 mit der Umhüllung 4 versehen wird, wobei letztere bevorzugt außenseitig fluchtend im Übergangsbereich 8 an die Ummantelung 6 anschließt. Diese Gestaltung hat einen je nach axialer Ausdehnung des Übergangsbereichs 8 gleichmäßigeren Übergang von der durch die Ummantelung 6 höheren Steifigkeit des Führungsdrahtschaftbereichs 5 zur niedrigeren Steifigkeit des distalen Abschnitts 3 zur Folge. Das zunächst vollständige Umgeben des Drahtkerns 2 mit der Ummantelung 6 kann z.B. durch entsprechende herkömmliche Extrusions- und Faserwickelvorgänge erfolgen. Es versteht sich, dass in alternativen, nicht gezeigten Ausführungsformen andere Arten des Übergangs zwischen der distalen Umhüllung 4 und der schaftseitigen Ummantelung 6 vorgesehen sein können, z.B. ein abrupter oder mehrstufiger Übergang.

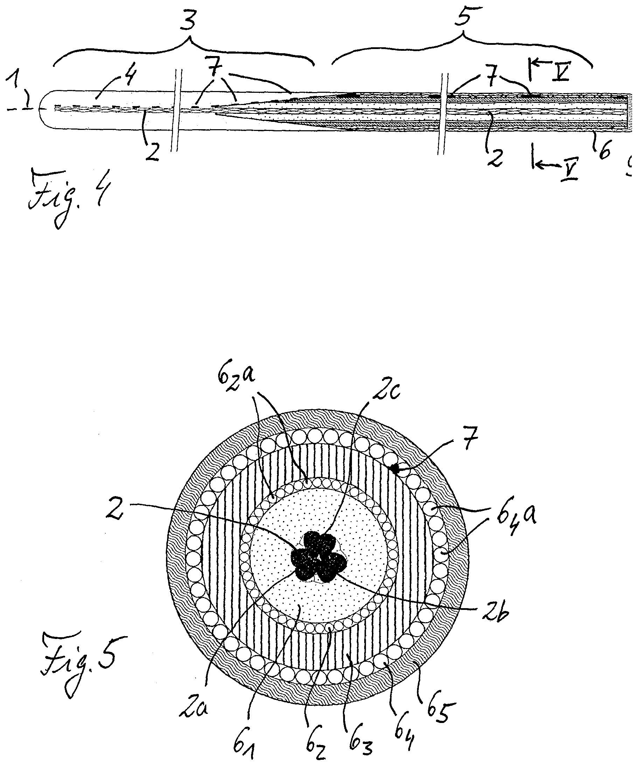

Die Fig. 4 und 5 veranschaulichen eine Variante des Führungsdrahtes der Fig. 1 bis 3, die sich von diesem lediglich in der Anzahl von Schichtlagen für die schaftseitige Ummantelung 6 unterscheidet, wobei zum leichteren Verständnis für identische und funktionell äquivalente Elemente gleiche Bezugszeichen verwendet sind und insoweit auf die obige Beschreibung zum Ausführungsbeispiel der Fig. 1 bis 3 verwiesen werden kann.

Wie aus den Fig. 4 und 5 zu erkennen, hat bei diesem Führungsdraht die Ummantelung 6 einen fünflagigen Schichtaufbau mit einer innersten Vollmaterial-Schichtlage 6₁, einer anschließenden ersten Faser-Schichtlage 6₂, einer daran anschließenden mittleren Vollmaterial-Schichtlage 6₃, einer anschließenden zweiten Faser-Schichtlage 6₄ und einer äußeren Vollmaterial-Schichtlage 6₅, die ihrerseits optional mit einer nicht gezeigten, z.B. hydrophilen außenseitigen Oberflächenbeschichtung versehen sein kann. Für die Vollmaterial-Schichtlagen 6₁, 6₃, 6₅ bzw. die Faser-Schichtlagen 6₂, 6₄ sind wiederum die gleichen Materialien verwendbar, wie oben für die betreffenden Schichtlagen zum Ausführungsbeispiel der Fig. 1 bis 3 angegeben. Für die drei Vollmaterial-Schichtlagen 6₁, 6₃, 6₅ können drei unterschiedliche Materialien verwendet werden, alternativ sind zwei oder alle drei der drei Vollmaterial-Schichtlagen aus dem gleichen Material gefertigt. Die beiden Faser-Schichtlagen 6₂, 6₄ können aus dem gleichen oder aus unterschiedlichen Fasermaterialien bestehen. Im gezeigten Beispiel sind beide Faser-Schichtlagen 6₂, 6₄ wiederum aus einer Monolage von in Umfangsrichtung berührend nebeneinanderliegenden Einzelfasern 6₂a, 6₄a gebildet, wobei gleiche oder unterschiedliche Fasermaterialien für die beiden Schichtlagen 6₂, 6₄ verwendet werden können. Bevorzugt unterscheiden sich die beiden Faser-Schichtlagen 6₂, 6₄ in ihren Faserwickelrichtungen und/oder ihren axialen Fasersteigungen. Dies ermöglicht bei gegebenem Material und gegebener Dicke für die verschiedenen Ummantelungs-Schichtlagen 6₁ bis 6₅ die Erzielung einer gewünscht hohen Biegesteifigkeit der Ummantelung 6.

Im Ausführungsbeispiel der Fig. 4 und 5 sind in gleicher Weise wie beim Ausführungsbeispiel der Fig. 1 bis 3 mehrere MR-sichtbare MR-Markerstreifenelemente in einer Linie längs der gesamten Führungsdrahtlänge angeordnet. Im distalen Abschnitt 3 sind sie mit Berührkontakt zum Drahtkern 2 in die einlagige Umhüllung 4 eingebettet, im Schaftabschnitt 5 sind sie in die zweite Faserschichtlage 6₄ integriert, d.h. die zugehörige Anordnungslinie erstreckt sich, wie aus Fig. 4 ersichtlich, im distalen Bereich axial entlang des Drahtkerns 2, dann im konischen Übergangsbereich 8 mit radialer Komponente bis zur zweiten Faser-Schichtlage 6₄ und dann wieder axial bis zum hinteren Führungsdrahtabschluss 9.

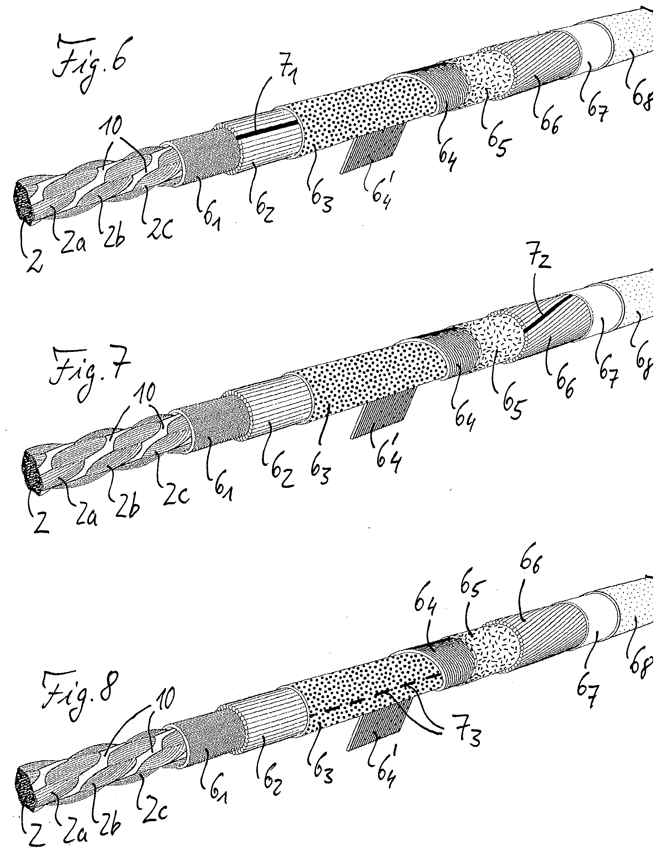

Die Fig. 6 bis 10 veranschaulichen verschiedene Varianten von Führungsdrähten mit auf unterschiedliche Weise in die Ummantelung integriertem MR-Marker, wobei nur beispielhaft eine jeweils achtlagige Ummantelung betrachtet wird und wobei wiederum für gleiche oder funktionell äquivalente Elemente gleiche Bezugszeichen verwendet sind wie zu den Beispielen der Fig. 1 bis 5, auf deren obige Beschreibung insoweit verwiesen werden kann.

Die Führungsdrähte der Fig. 6 bis 9 weisen jeweils einen Drahtlitzenkern 2 aus drei miteinander verseilten Einzeldrahtlitzen 2a, 2b, 2c auf, der im Schaftabschnitt von einer biegesteiferen Ummantelung umgeben ist, deren achtlagiger Schichtaufbau fünf Vollmaterial- und drei Faser-Schichtlagen umfasst. Im Einzelnen sind dies von innen nach außen eine den Drahtkern 2 mit Berührkontakt umgebende, erste Vollmaterial-Schichtlage 6₁, eine erste Faser-Schichtlage 6₂, eine zweite Vollmaterial-Schichtlage 6₃, eine zweite Faser-Schichtlage 6₄, eine dritte Vollmaterial-Schichtlage 6₅, eine dritte Faser-Schichtlage 6₆, eine vierte Vollmaterial-Schichtlage 6₇ und eine äußere, fünfte Vollmaterial-Schichtlage 6₈. Wie zeichnerisch angedeutet, bestehen die fünf Vollmaterial-Schichtlagen vorzugsweise aus fünf unterschiedlichen Materialien, alternativ auch nur aus zwei bis vier unterschiedlichen Materialien, die sich z.B. in ihrer Härte bzw. Sprödigkeit und/oder in ihrer Zähigkeit bzw. Festigkeit unterscheiden, oder es bestehen alle fünf Vollmaterial-Schichtlagen aus dem gleichen Material. So lassen sich Schichtlagen unterschiedlicher Härten und Festigkeiten beliebig miteinander kombinieren, um ein gewünschtes Steifigkeitsverhalten und andere gewünschte Eigenschaften für den Führungsdraht bereitzustellen.

Es versteht sich, dass in alternativen Ausführungsformen eine beliebige andere Anzahl von Vollmaterial-Schichtlagen gewählt werden kann. Ebenso können die Vollmaterial-Schichtlagen statt mit den gezeigten drei mit einer beliebigen anderen Anzahl von Faser-Schichtlagen kombiniert sein. In den gezeigten Ausführungen befindet sich jede Faser-Schichtlage zwischen zwei angrenzenden Vollmaterial-Schichtlagen, in alternativen Ausführungen ist aber auch jede beliebige andere Abfolge von Vollmaterial-Schichtlagen und Faser-Schichtlagen verwendbar, z.B. auch zwei aufeinanderfolgende Faser-Schichtlagen.

Hinsichtlich der Materialen für die Vollmaterial- und die Faser-Schichtlagen kann auf die Ausführungen zu den Beispielen der Fig. 1 bis 5 verwiesen werden. Dabei können die Materialien der Vollmaterial-Schichtlagen bei Bedarf mit zusätzlichen Füllstoffen versehen sein, wie dem Fachmann an sich bekannt. Die Faser-Schichtlagen können sich bei Bedarf zudem in ihren Wickelrichtungen und/oder Fasersteigungen unterscheiden. So besitzt bei der gezeigten Realisierung der Fig. 6 bis 10 die erste Faser-Schichtlage 6₂ einen axialen Faserverlauf, während die Fasern der zweiten Faser-Schichtlage 6₄ schraubenförmig gewickelt sind, wie anhand eines hierzu in diesen Schnittaufbaudarstellungen noch gezeigten Wickelrestes 6₄' ergänzend symbolisiert. Die dritte Faser-Schichtlage 6₆ ist ebenfalls schraubenförmig gewickelt, jedoch mit deutlich größerer Steigung.

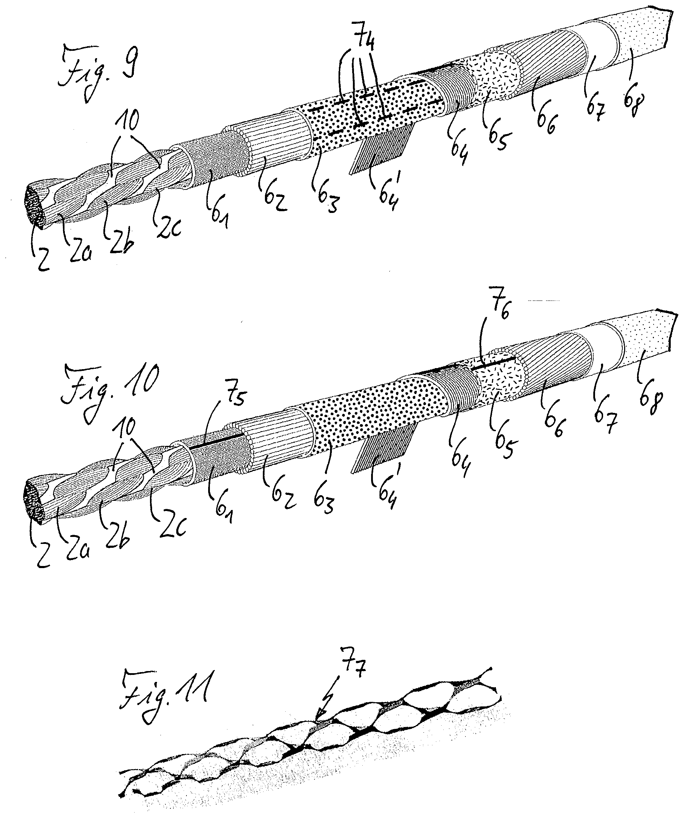

Die Fig. 6 bis 10 veranschaulichen stellvertretend für zahlreiche weitere Möglichkeiten, den Führungsdraht im Bereich der Ummantelung MR-sichtbar zu machen, einige Varianten, bei denen linienförmige MR-Markerelemente über die axiale Länge der Ummantelung in letztere integriert sind. Im Beispiel der Fig 6 ist dazu eine der Fasern der ersten Faser-Schichtlage 6₂ aus einem MR-sichtbaren Material gefertigt und repräsentiert dadurch einen sich in dieser Schichtlage 6₂ axial erstreckenden MR-Markerstreifen 7₁. Im Beispiel von Fig. 7 ist analog eine der Fasern der dritten Faser-Schichtlage 6₆ aus einem MR-sichtbaren Material gefertigt und repräsentiert dadurch einen sich in dieser Schichtlage 6 schraubenförmig erstreckenden MR-Markerstreifen 7₂. Im Beispiel von Fig. 8 sind mehrere kürzere MR-Markerstreifen 7₃ längs einer axial verlaufenden Linie mit Abstand voneinander in die zweite Vollmaterial-Schichtlage 6₃ eingebettet bzw. an deren Außenseite angeordnet. Im Beispiel von Fig. 9 sind zwei in Umfangsrichtung beabstandete Reihen von solchen kürzeren, voneinander axial beabstandeten MR-Markerstreifen 7₄ jeweils längs einer axial verlaufenden Linie in die zweite Vollmaterial-Schichtlage 6₃ eingebettet bzw. an deren Außenseite angeordnet. Im Beispiel von Fig. 10 sind zwei über die Länge der Ummantelung durchgehende MR-Markerstreifen 7₅ und 7₆ axial verlaufend in die erste Vollmaterial-Schichtlage 6₃ bzw. die dritte Vollmaterial-Schichtlage 6₅ eingebettet oder an deren Außenseite angeordnet. Es versteht sich, dass je nach Bedarf und Anwendungsfall weitere MR-Markerelemente in die diversen Schichtlagen der Ummantelung eingebracht sein können und die in den Fig. 6 bis 10 gezeigten Alternativen beliebig kombiniert werden können.

Zusätzlich oder alternativ zu einer solchen Integration eines oder mehrerer MR-Markerelemente in die Ummantelung kann der MR-Marker erfindungsgemäß auch MR-sichtbares Material umfassen, das in Zwischenräume eines als Litze realisierten Drahtkerns eingebracht ist. Fig. 11 zeigt ein entsprechendes MR-Markergerüst 7₇, wie es vorliegt, wenn ein Zwischenraum 10, wie er bei den Führungsdrahtvarianten der Fig. 6 bis 10 zwischen den Einzeldrähten innerhalb der ersten Vollmaterial-Schichtlage 6₁ der Ummantelung verbleibt, in dieser Weise mit MR-sichtbarem Material gefüllt ist. Zur besseren Erkennbarkeit sind alle anderen Führungsdrahtkomponenten weggelassen.

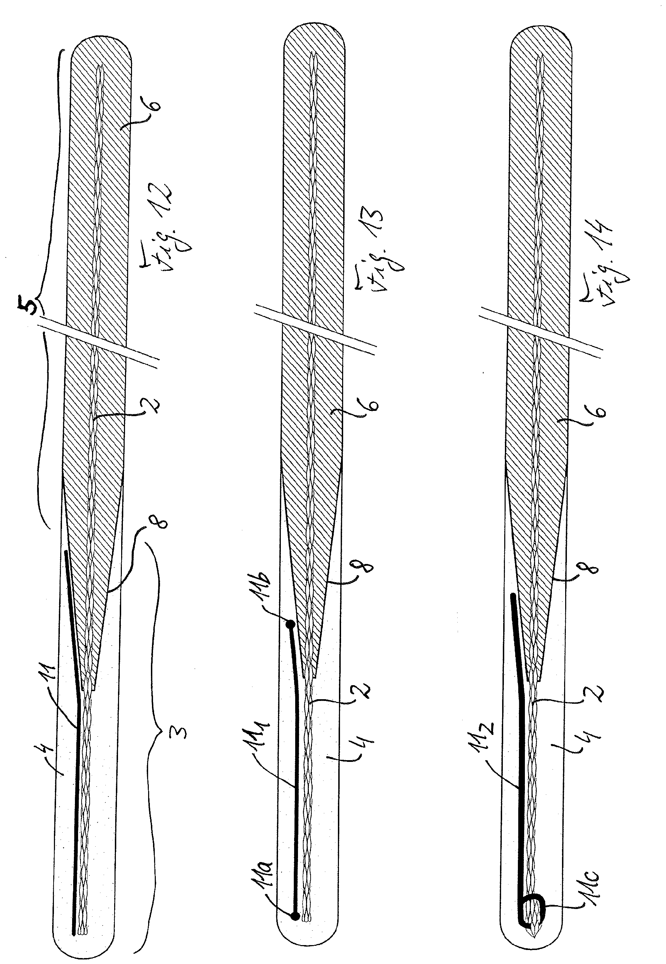

Die Fig. 12 bis 38 veranschaulichen weitere Ausführungsformen der Erfindung, speziell solche, bei denen ein draht- oder rohrförmiges Hilfselement aus einem MR-sichtbaren, nichtmagnetischen Material, z.B. einem entsprechenden Metallmaterial, gezielt in einem distalen Führungsdrahtabschnitt angeordnet ist, um dessen MR-Sichtbarkeit zu verstärken und/oder dessen elastische Eigenschaften gezielt zu beeinflussen. Soweit im Folgenden nichts Anderes gesagt, können die zu den Fig. 12 bis 38 erwähnten Führungsdrähte in ihren Bestandteilen, Funktionen und Eigenschaften den oben zu den Fig. 1 bis 11 erwähnten Führungsdrähten entsprechen, so dass insoweit auf deren obige Erläuterungen verwiesen werden kann. Außerdem sind auch hier wiederum zum leichteren Verständnis für identische und funktionell äquivalente Elemente jeweils gleiche Bezugszeichen verwendet.

Der in Fig. 12 verkürzt gezeigte Führungsdraht weist wie der Führungsdraht der Fig. 1 bis 3 einen Drahtkern 2 auf, der in einem vorderen, distalen Führungsdrahtabschnitt 3 von einer biegeweicheren Umhüllung 4 und in einem daran anschließenden Schaftabschnitt 5 von einer biegesteiferen Ummantelung 6 umgeben ist, wie oben zu den Fig. 1 bis 3 näher erläutert. Dabei ist auch in Fig. 12 ein Beispiel gezeigt, bei dem die aus dem biegeweicheren Material bestehende distale Umhüllung 4 in axialer Richtung unter Bildung eines stetigen, konischen Übergangs 8 an die Schaftummantelung 6 anschließt.

Zusätzlich weist der Führungsdraht von Fig. 12 ein drahtförmiges Hilfselement 11 auf, das in die distale Umhüllung 4 eingebettet ist und sich im Wesentlichen axial neben dem Drahtkern 2 von dessen distalem Ende bis in den konischen Übergangsbereich 8 der Umhüllung 4 erstreckt, wobei es im konischen Übergangsbereich 8 dem kegelstumpfförmigen Konus mit einer entsprechenden Radialkomponente zusätzlich zur Axialkomponente folgt. Der Hilfselementdraht 11 besteht aus einem MR-sichtbaren, nichtmagnetischen Material, vorzugsweise einem entsprechenden Metallmaterial, wie einer superelastischen Nickel-Titan-Legierung, oder einem MR-sichtbaren Kunststoffmaterial, z.B. einem Kunststoffmaterial, das mit einem MR-Markermaterial dotiert ist. Mit diesem Hilfselementdraht 11 lässt sich die MR-Sichtbarkeit speziell des distalen Endabschnitts 3 des Führungsdrahtes verstärken. Außerdem lassen sich die elastischen Eigenschaften des distalen Führungsdrahtabschnitts 3 in gezielter Weise beeinflussen, beispielsweise dahingehend, eine gewünschte Formgebung für das distale Spitzenende des Führungsdrahtes bereitzustellen, wie eine J-Spitzenform oder eine andere Abwinklung des distalen Führungsdrahtendes.

Die Ummantelung 6 kann beim Führungsdraht von Fig. 12 je nach Bedarf einen herkömmlichen einlagigen Schichtaufbau oder einen mehrlagigen Schichtaufbau aufweisen, wie er oben zu den Fig. 1 bis 11 beschrieben ist.

Der in Fig. 13 gezeigte Führungsdraht unterscheidet sich von demjenigen der Fig. 12 in einer Modifikation des Hilfselementdrahtes. Speziell ist hier ein Hilfselementdraht 11₁ verwendet, der an beiden Enden mit je einer pilzkopfförmigen Verdickung 11a, 11b versehen ist. Diese endseitigen Verdickungen 11a, 11b unterstützen eine sichere Fixierung des Hilfselementdrahtes 11₁ in der einbettenden, umschließenden distalen Umhüllung 4.

Der in Fig. 14 gezeigte Führungsdraht beinhaltet als Variante desjenigen von Fig. 12 einen modifizierten Hilfselementdraht 11₂, an dessen distalem Ende eine Drahtbiegung bzw. Drahtschleife 11c ausgebildet ist, mit welcher er wie gezeigt am distalen Ende des Drahtkerns 2 befestigt ist. Dazu greift die Drahtbiegung 11c in das Fasergeflecht- oder Litzenmaterial hinein und durch dieses hindurch, aus dem der Drahtkern 2 gebildet ist. Auf diese Weise ist das drahtförmige Hilfselement 11₂ mit seinem distalen Ende sicher am distalen Ende des Drahtkerns 2 gehalten.

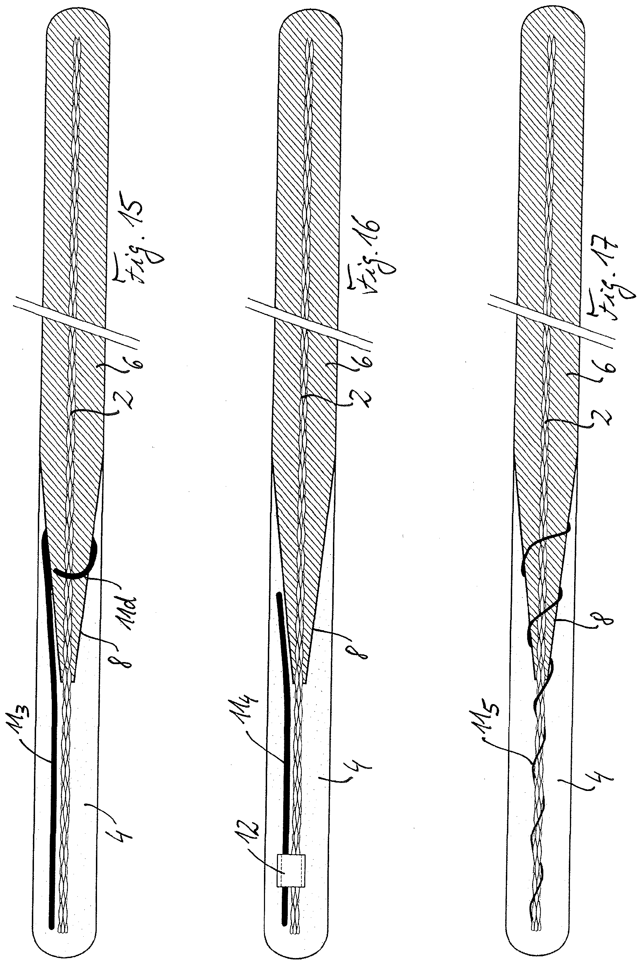

Fig. 15 zeigt eine Führungsdrahtvariante mit einem Hilfselementdraht 11₃, der im Unterschied zum Führungsdraht von Fig. 12 an seinem proximalen Ende in eine Drahtbiegung bzw. Drahtschleife 11d gebogen ist, mit welcher er sich um den Kegelstumpf des Übergangskonus 8 legt, was wiederum eine sichere Fixierung des Hilfselementdrahts 11₃ in der einbettenden Kernumhüllung 4 fördert.

Bei einer in Fig. 16 gezeigten Führungsdrahtvariante ist ein Hilfselementdraht 11₄ nach Art von Fig. 12 vorgesehen, wobei hier der Hilfselementdraht 11₄ mittels eines zusätzlichen Halterings 12 am Drahtkern 2 fixiert ist, um ihn sicher in der einbettenden Kernumhüllung 4 zu halten. Alternativ können auch mehrere derartige Halteringe axial nebeneinander vorgesehen sein. Der jeweilige Haltering 12 kann z.B. aus Gold, Platin, Wolfram oder einem anderen Metall bestehen und dadurch als MR-Marker und als röntgensichtbarer Marker fungieren.

Fig. 17 zeigt als weitere Variante einen Führungsdraht mit einem Hilfselementdraht 11₅, der sich schraubenlinienförmig um den Drahtkern 2 und den Übergangskonus 8 herum erstreckt. Auch diese Realisierung trägt zu einem sicheren Halt dieses Hilfselementdrahtes 11₅ in der einbettenden Kernumhüllung 4 bei.

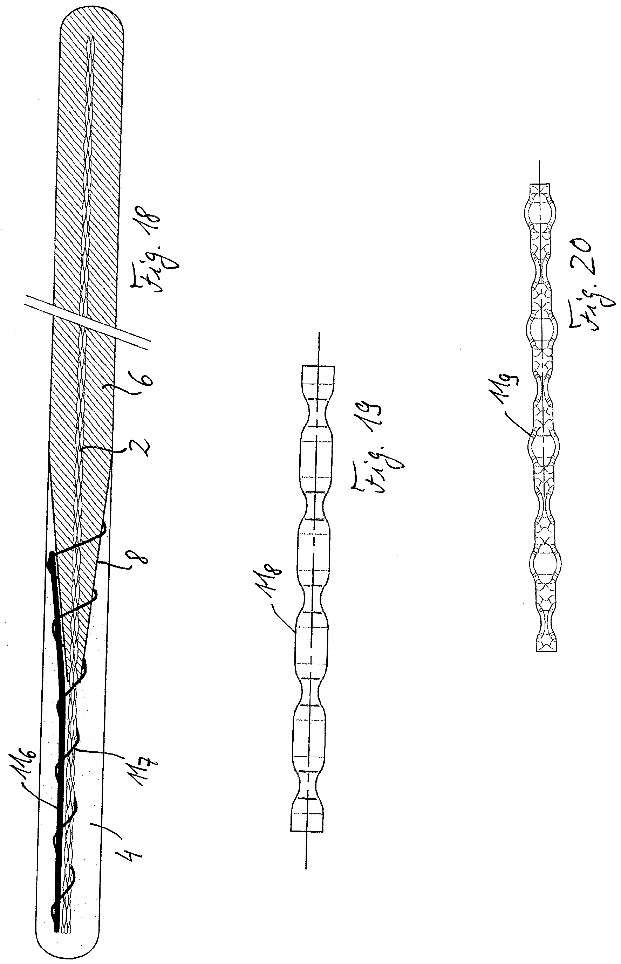

Fig. 18 zeigt eine Führungsdrahtvariante, die einer Kombination der Hilfselemente der Fig. 12 und 17 entspricht. Als Hilfselement zur Verstärkung der MR-Sichtbarkeit des distalen Führungsdrahtabschnitts dient dort ein lineares Drahtstück 11₆ nach Art des Drahtstücks 11 von Fig. 12 in Kombination mit einem sich schraubenlinienförmig um den Drahtkern 2 und das lineare Drahtstück 11₆ herum erstreckenden Drahtstück 11₇. Dies kombiniert entsprechend die Vorteile der in den Fig. 12 und 17 gezeigten Varianten.

Für die Realisierung der als MR-sichtbare Hilfselemente 11 bis 11₇ in den Führungsdrähten der Fig. 12 bis 18 vorgesehenen Drahtstücke bieten sich je nach Bedarf und Anwendungsfall verschiedene Alternativen an. So sind beispielsweise massive Drahtstücke oder Drahtlitzen mit über die Länge gleichbleibendem, z.B. kreisrundem Querschnitt einsetzbar. Alternativ kann der Drahtquerschnitt über die Drahtlänge hinweg zur Erzielung gewünschter Biegeeigenschaften des Führungsdrahtes modifiziert sein. So weist in einem Ausführungsbeispiel der Hilfselementdraht einen in distaler Richtung konisch abnehmenden Durchmesser auf, wodurch sich eine graduell abnehmende Biegesteifigkeit für den distalen Führungsdrahtendabschnitt einstellen lässt.

Fig. 19 zeigt eine Realisierung eines Hilfselementdrahtstücks 11₈ mit in axialer Richtung alternierend zwischen einem minimalen Durchmesserwert und einem maximalen Durchmesserwert variierendem Drahtdurchmesser. Dieser Hilfselementdraht 11₈ lässt sich beispielsweise durch abschnittweises umfangseitiges Schleifen eines massiven Rohdrahtstücks mit konstantem Durchmesser fertigen. Fig. 20 zeigt einen alternativ verwendbaren Hilfselementdraht 11₉ mit in axialer Richtung periodisch variablem Durchmesser, der sich beispielsweise durch einen entsprechenden Drahtpressvorgang herstellen lässt.

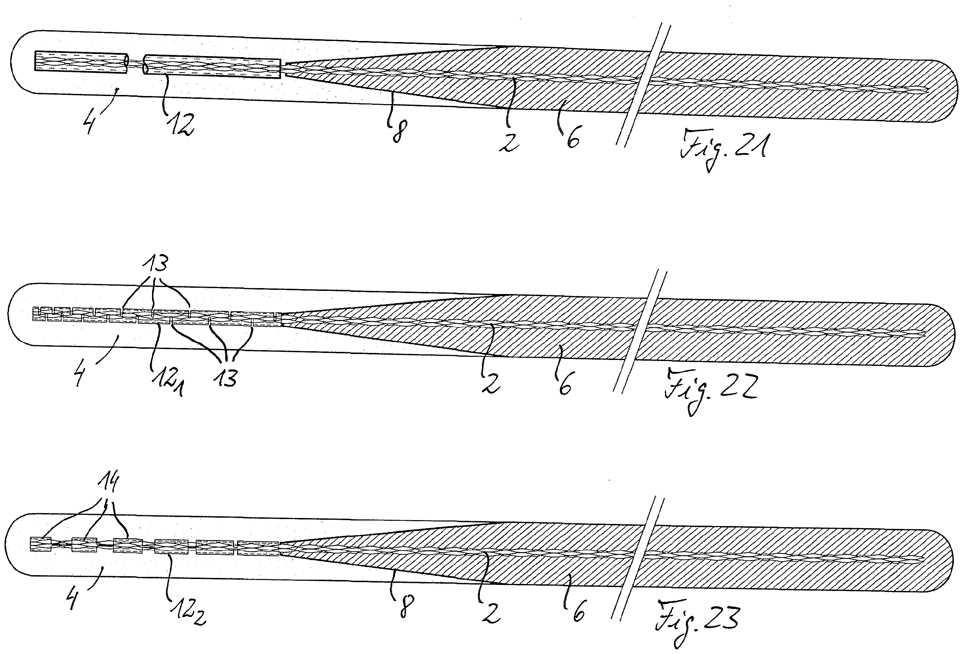

Anstelle oder zusätzlich zu einem drahtförmigen Hilfselement, wie vorstehend erläutert, kann ein MR-sichtbares hülsen- bzw. rohrförmiges Hilfselement aus einem nichtmagnetischen Material in einem distalen Führungsdrahtabschnitt vorgesehen sein, um die MR-Sichtbarkeit dieses Abschnitts zu verstärken und bei Bedarf dessen elastische Eigenschaften, d.h. Biegeeigenschaften, in einer gewünschten Weise zu beeinflussen. Dazu umgibt das rohrförmige Hilfselement den Drahtkern im betreffenden distalen Abschnitt. Wie die drahtförmigen Hilfselemente können auch die rohrförmigen Hilfselemente aus einem geeigneten MR-sichtbaren Kunststoffmaterial oder nichtmagnetischen Metallmaterial gebildet sein. Je nach Bedarf können die drahtförmigen oder rohrförmigen Hilfselemente aus entsprechendem Draht-, Litzen-, Faden-, Geflecht- oder Rohrmaterial gefertigt sein. Die Fig. 21 bis 38 veranschaulichen verschiedene Führungsdrahtvarianten mit derartigen Hilfselementhülsen bzw. Hilfselementrohren im distalen Führungsdrahtabschnitt.

Fig. 21 zeigt wiederum in verkürzter Darstellung einen Führungsdraht mit einem solchen MR-sichtbaren Hilfselementrohr 12 statt des Hilfselementdrahtes 11 von Fig. 12, wobei der Führungsdraht von Fig. 21 ansonsten demjenigen von Fig. 12 entspricht. Das Hilfselementrohr 12 umgibt, wie gezeigt, den Drahtkern 2 in dessen distalem Endbereich bis zum Übergangskonus 8 von Umhüllung 4 und Ummantelung 6. In diesem Beispiel besteht das Hilfselementrohr 12 aus einem einheitlichen Rohrstück. Das Hilfselementrohr 12 ist wie gezeigt in die biegeweiche distale Kernumhüllung 4 eingebettet, wobei das Einbettungsmaterial auch den Ringspalt zwischen Drahtkern 2 und umgebenden Hilfselementrohr 12 füllt.

Fig. 22 veranschaulicht eine Variante des Führungsdrahtes von Fig. 21 mit einem modifizierten Hilfselementrohr 12₁, das aus einem Rohrstück gefertigt ist, in welches umfangsseitig gegenüberliegende und axial gegeneinander versetzte Einschnitte 13 in radialer Richtung eingebracht sind, die sich jeweils etwa bis zur Rohrmitte erstrecken, d.h. mit in etwa halber Umfangslänge. Zur gezielten Beeinflussung des Biegeverhaltens des Führungsdrahtes in seinem distalen Endabschnitt können die Einschnitte 13 geeignet gewählt werden, z.B. wie gezeigt mit in distaler Richtung abnehmenden axialen Abstand voneinander, so dass sich für das Hilfselementrohr 13 und folglich für den distalen Führungsdrahtabschnitt insgesamt eine in distaler Richtung abnehmende Biegesteifigkeit ergibt.

Fig. 23 zeigt eine Führungsdrahtvariante, bei welcher ein Hilfselementrohr 12₂ aus einzelnen Rohrabschnitten 14 vorgesehen ist, die lose oder in nicht gezeigter Weise miteinander verbunden in die distale Umhüllung 4 eingebettet sind und den Drahtkern 2 umgeben. Im gezeigten Beispiel sind zur Erzielung einer in distaler Richtung abnehmenden Biegesteifigkeit die Rohrabschnitte 14 mit in distaler Richtung abnehmender Länge und zunehmendem Abstand voneinander angeordnet.

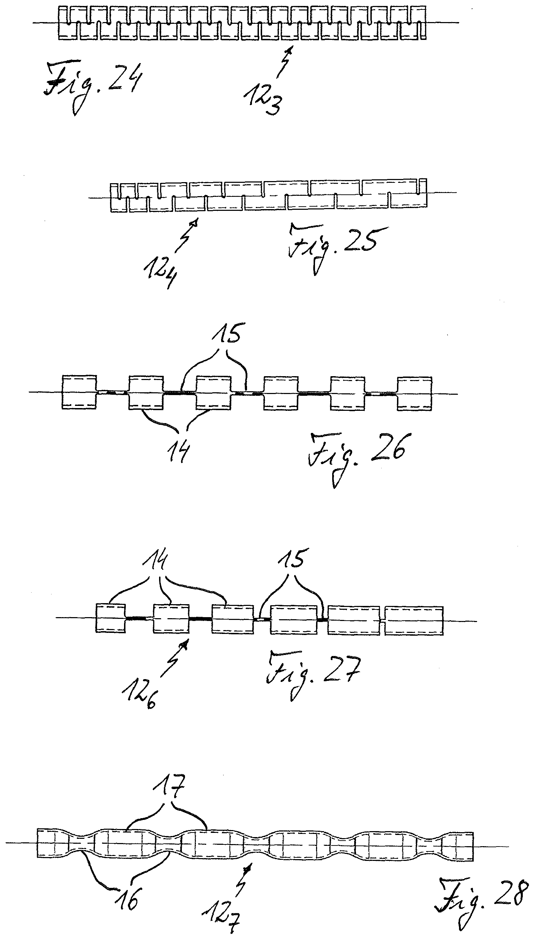

Die Fig. 24 bis 29 zeigen verschiedene mögliche Realisierungen von erfindungsgemäß verwendbaren Hilfselementrohren, die hinsichtlich Beeinflussung der Biegesteifigkeit modifiziert sind. So zeigt Fig. 24 ein Hilfselementrohr 12₃, das wie das Hilfselementrohr 12₁ im Führungsdraht von Fig. 22 mit axial versetzten und umfangsseitig alternierend gegenüberliegenden Einschnitten 13 versehen ist. In diesem Beispiel sind die Einschnitte 13 mit gleichem axialem Abstand voneinander eingebracht, so dass sie eine gleichmäßige Erhöhung der Flexibilität, d.h. eine gleichmäßige Verringerung der Biegesteifigkeit, für dieses Hilfselementrohr 12₃ ergeben. Alternativ zeigt Fig. 25 ein Hilfselementrohr 12₄ ähnlich dem von Fig. 24, wobei jedoch hier die Einschnitte 13 mit in distaler Richtung abnehmendem axialem Abstand voneinander eingebracht sind. Dies führt zu einer verringerten Biegesteifigkeit dieses Hilfselementrohrs 12₄ und damit des distalen Führungsdrahtabschnitts, in welchem das Hilfselementrohr 12₄ eingesetzt wird. Dies entspricht der Verwendung des analogen Hilfselementrohrs 12₁ im Führungsdraht von Fig. 22.

Fig. 26 zeigt ein Hilfselementrohr 12₅, das ähnlich wie das Hilfselementrohr 12₂ im Führungsdraht von Fig. 23 aus einzelnen Ringsegmenten 14 gebildet ist, die in axialer Richtung aufeinanderfolgend angeordnet sind. In diesem Beispiel sind die Ringsegmente 14 über schmale axiale Stege 15 miteinander verbunden, so dass das Hilfselementrohr 12₅ ein einteiliges Bauteil bildet, das beispielsweise durch einen geeigneten Schneidprozess aus einem homogenen Rohrstück herausgeschnitten sein kann. Es ergibt sich dadurch wiederum eine verringerte Biegesteifigkeit für das Hilfselementrohr 12₅ im Vergleich zum ungeschnittenen einteiligen Rohrstück.

Fig. 27 zeigt ein gegenüber Fig. 26 modifiziertes Hilfselementrohr 12₆, bei dem die Rohrsegmente bzw. Rohrabschnitte 14 in distaler Richtung mit sukzessiv abnehmender Länge und zunehmendem axialem Abstand voneinander angeordnet sind. Dieses Hilfselementrohr 12₆ entspricht somit dem im Führungsdraht von Fig. 23 eingesetzten Hilfselementrohr 12₂.

Fig. 28 zeigt ein modifiziertes Hilfselementrohr 12₇, das abschnittweise alternierend Bereiche 16 mit geringerem Durchmesser und Bereiche 17 mit größerem Durchmesser aufweist. Selbstverständlich sind die Bereiche mit dem niedrigeren Durchmesser 16 so bemessen, dass dort der Rohrdurchmesser noch größer als der Durchmesser des Drahtkerns ist, so dass der betreffende distale Endabschnitt des Drahtkerns im Hilfselementrohr 12₇ aufgenommen werden kann.

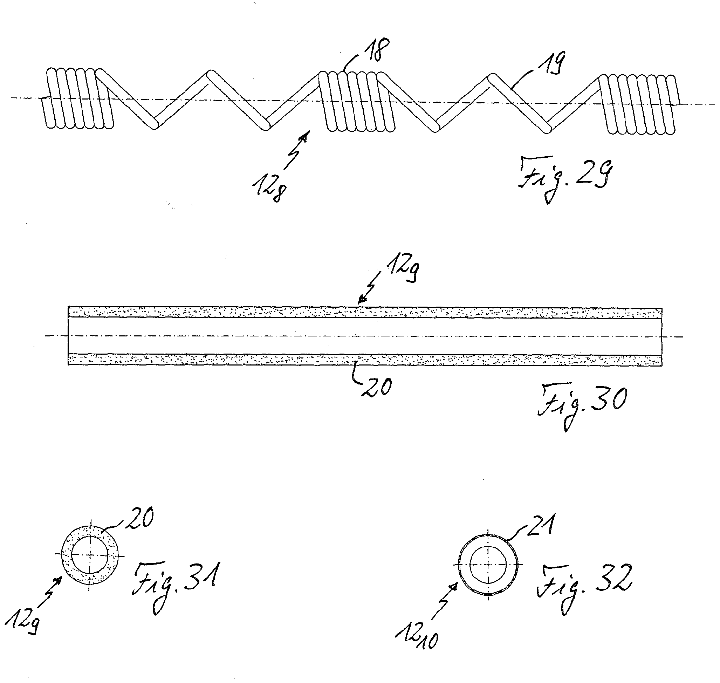

Fig. 29 zeigt ein Hilfselementrohr 12₈, das durch ein Schraubenfederstück gebildet ist. Dabei wechseln zur gezielten Beeinflussung des Biegesteifigkeitsverhaltens Bereiche 18, in denen benachbarte Wicklungen aneinander anliegen, mit biegeweicheren Bereichen 19 ab, in denen die Wicklungen auseinandergezogen und dadurch axial voneinander beabstandet sind. Es versteht sich, dass alternativ beliebige andere Schraubenfedergestaltungen für diese Art von Hilfselementrohr möglich sind, z.B. als einheitliches Schraubenfederstück oder als ein Schraubenfederstück mit in distaler Richtung sukzessiv oder schrittweise zunehmendem Wicklungsabstand.

In denjenigen Führungsdrahtvarianten, bei denen das draht- oder rohrförmige Hilfselement in axialer Richtung nicht einheitlich ist, sondern eine Variation aufweist, wie beispielsweise die Varianten mit schraubenlinienförmigem Hilfselementdraht oder die Varianten mit einem Hilfselementrohr aus axial aufeinanderfolgenden Rohrsegmenten bzw. Rohrabschnitten oder aus Schraubenfederabschnitten mit unterschiedlichem Wicklungsabstand, kann diese Eigenschaft einer entsprechend in axialer Richtung variierenden Sichtbarkeit bei MR-Anwendungen oder auch bei Röntgenanwendungen für Abstandsmessungen benutzt werden, d.h. aufgrund dieser Eigenschaft lässt sich auf sehr einfache Weise die genaue Position des mit dem draht- oder rohrförmigen Hilfselement versehenen distalen Führungsdrahtabschnitts detektieren und für Abstandsmessungen ausnutzen.

Die Fig. 30 und 31 zeigen ein rohrförmiges Hilfselement 12₉, das aus einem einheitlichen Rohrstück gebildet ist, welches aus einem mit einem MR-Markermaterial MR-sichtbar dotierten Grundmaterial 20 besteht. Dies gewährleistet eine gute MR-Sichtbarkeit eines distalen Führungsdrahtabschnitts, wenn dieses Hilfselementrohr 12₉ dort z.B. nach Art des Hilfselementrohrs 12 von Fig. 21 eingesetzt ist.

Fig. 32 veranschaulicht eine Variante des Hilfselementrohrs 12₉ der Fig. 30 und 31 in Form eines Hilfselementrohrs 12₁₀, das eine oberflächliche Dotierung bzw. Beschichtung 21 aus einem MR-Markermaterial aufweist, anstelle der Volumendotierung im Beispiel der Fig. 30 und 31.

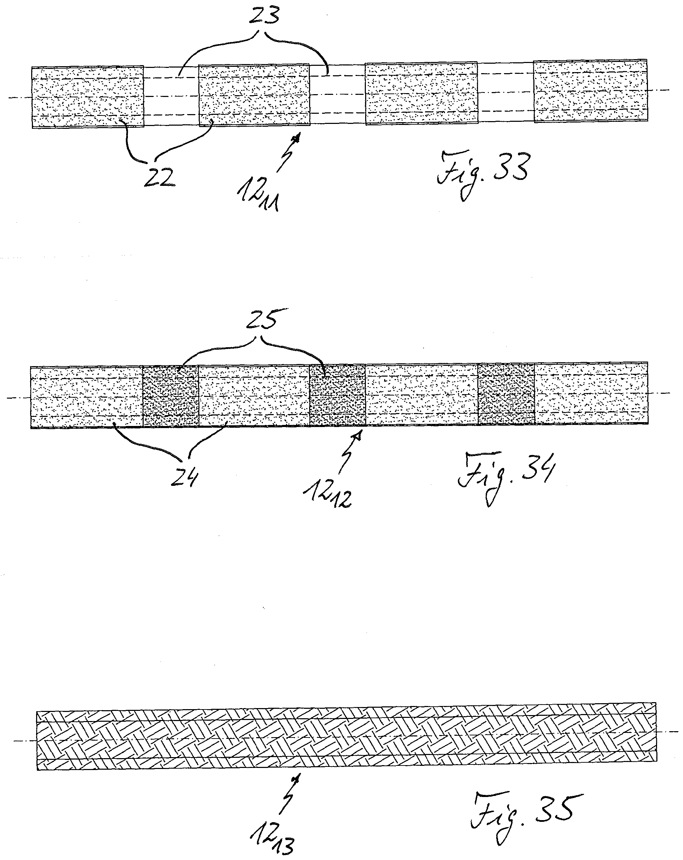

Fig. 33 veranschaulicht eine Variante des Hilfselementrohrs 12₁₀ in Form eines Hilfselementrohrs 12₁₁, das an seiner Außenseite statt ganzflächig nur partiell mit der oberflächlichen MR-Markerdotierung bzw. MR-Markerbeschichtung versehen ist. Speziell beinhaltet die partielle Beschichtung in diesem Beispiel einzelne MR-sichtbar beschichtete bzw. dotierte Rohrabschnitte 22, die mit axialem Abstand voneinander angeordnet sind, wobei zwischen ihnen undotierte bzw. unbeschichtete Bereiche 23 verbleiben.

Fig. 34 veranschaulicht eine Abwandlung des Hilfselementrohrs 12₁₁ von Fig. 33 in Form eines Hilfselementrohrs 12₁₂, bei dem sich in axialer Richtung Bereiche 24 einer ersten oberflächlichen MR-Markerbeschichtung bzw. MR-Markerdotierung in axialer Richtung mit Bereichen 25 einer von der ersten verschiedenen zweiten MR-Markerdotierung bzw. MR-Markerbeschichtung abwechseln. Mit den Varianten der Fig. 33 und 34 sind bei Bedarf wieder die oben erwähnten Abstandsmessungen bzw. Führungsdraht-Positionsbestimmungen unter Ausnutzung der in axialer Richtung variierenden MR-Sichtbarkeit der Hilfselementrohre 12₁₁, 12₁₂ möglich.

Wie erwähnt, kann das jeweilige Hilfselementrohr aus einer superelastischen Nickel-Titan-Legierung, einem anderen nichtmagnetischen Metall oder aus einem MR-sichtbar dotierten oder alternativ nicht dotierten Kunststoffmaterial bestehen. Dabei ist anstelle eines einheitlichen, homogenen Aufbaus des Hilfselementrohrs aus dem betreffenden Material auch eine Herstellung aus einem Geflecht derartiger nichtmagnetischer Metall- oder Kunststoffmaterialien möglich. Fig. 35 zeigt ein entsprechendes Hilfselementrohr 12₁₃, das aus einem solchen Schlauchgewebematerial gebildet ist.

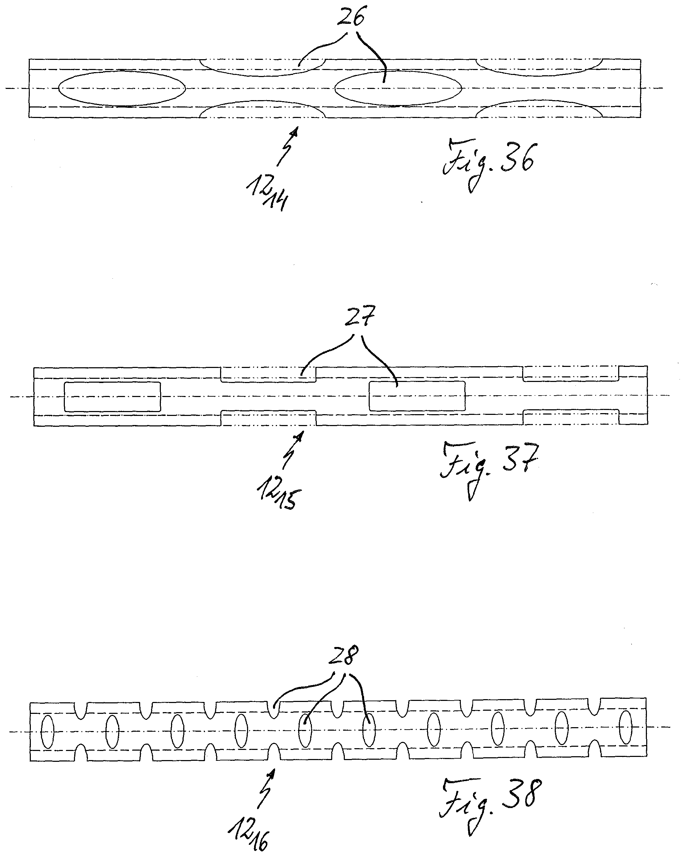

Die Fig. 36 bis 38 veranschaulichen weitere vorteilhafte Gestaltungen erfindungsgemäß verwendbarer Hilfselementrohre, die mit verschiedenen Einschnitten versehen sind, mit denen sich die Biegesteifigkeit in gewünschter Weise beeinflussen lässt und sich zudem eine bessere Verbindung des Hilfselementrohrs mit dem in ihr aufgenommenen distalen Abschnitt des Drahtkerns erzielen lässt. Speziell zeigt Fig. 36 ein Hilfselementrohr 12₁₄, das mit ovalen Einschnitten 26 versehen ist, die jeweils paarweise in Umfangsrichtung gegenüberliegend und in axialer Richtung aufeinanderfolgend um 90° versetzt in den Rohrmantel eingebracht sind.

Fig. 37 zeigt ein ähnliches Hilfselementrohr 12₁₅, bei dem rechteckförmige Einschnitte 27 anstelle der ovalen Einschnitte 26 im Beispiel der Fig. 36 in den Rohrmantel eingebracht sind.

Fig. 38 zeigt ein Hilfselementrohr 12₁₆, bei dem in den Rohrmantel ovale Einschnitte 28 eingebracht sind, die sich quer zur Axialrichtung des Rohres 12₁₆ statt wie die ovalen Einschnitte 26 im Beispiel von Fig. 36 in Rohraxialrichtung erstrecken.

Wie die zahlreichen Beispiele anhand der Fig. 12 bis 38 deutlich machen, stellt die Erfindung eine große Vielfalt an MR-sichtbaren, draht- oder rohrförmigen Hilfselementen aus nichtmagnetischem Material zur Verfügung, mit denen ein distaler Führungsdrahtabschnitt bestückt sein kann, um seine MR-Sichtbarkeit zu verstärken und/oder seine Biegeeigenschaften in einer gewünschten Weise zu beeinflussen. Ergänzend sei erwähnt, dass bei Bedarf die Schaftummantelung 4 und/oder die distale Umhüllung 2 mit einem Markierungsmuster versehen sein kann, die dazu benutzt werden kann, Längs- und/oder Drehbewegungen des Führungsdrahtes zu detektieren und/oder Längen abzumessen. Hierzu können insbesondere Markierungsmuster verwendet werden, wie sie in den Patentschriften DE 102 43 261 B4 und DE 102 55 030 B4 sowie der Offenlegungsschrift WO 2009/112048 A1 beschrieben sind.

Die bislang zu den Figuren beschriebenen Ausführungsformen der Erfindung stellen Führungsdrähte dar, die sich nicht nur für MR-Anwendungen verwenden lassen, sondern auch für HF-Anwendungen. Für die letztgenannten Anwendungen ist es nicht zwingend erforderlich, dass der Führungsdraht einen MR-Marker aufweist und sein Drahtkern aus einem MR-unsichtbaren Material besteht. Vielmehr kann in diesem Fall der Drahtkern beispielsweise auch aus einer superelastischen Nickel-Titan-Legierung bestehen, die von der mehrlagigen Schaftummantelung 6 aus elektrisch isolierendem Material umgeben ist, die für eine ausreichende elektrische Isolation sorgt. Beispielsweise kann der HFfähige Führungsdraht ein solcher sein, der entsprechend den Fig. 1 bis 3 aufgebaut ist, wobei der Drahtkern 2 in diesem Fall aus einer Nickel-Titan-Legierung bestehen kann. Alternativ kann der Drahtkern 2 auch aus einem hochfesten, biegesteifen Material bestehen, wie einem faserverstärkten und insbesondere einem kohlefaserverstärkten Kunststoffmaterial. Bei dem HF-fähigen Führungsdraht kann in entsprechenden Ausführungsformen der Drahtkern 2 biegesteifer sein als die mehrlagige Schaftummantelung.

Wie die gezeigten und oben beschriebenen Ausführungsbeispiele deutlich machen, stellt die Erfindung einen sehr vorteilhaften Führungsdraht mit einer mehrlagigen Ummantelung eines Drahtkerns bereit, mit der das Steifigkeitsverhalten des Führungsdrahtes in einer gewünschten Weise einstellbar ist. Zudem eignet sich der erfindungsgemäße Führungsdraht sehr gut für MR-Anwendungen und/oder für HF-Anwendungen.

## Patentansprüche

1. Medizinischer Führungsdraht mit
- einem Drahtkern (2) aus einem MR-unsichtbaren Material,
- einer den Drahtkern wenigstens abschnittsweise und mit Berührkontakt umgebenden Ummantelung (6), die einen mehrlagigen Schichtaufbau besitzt, der wenigstens zwei Vollmaterial-Schichtlagen (6₁, 6₃) und wenigstens eine Faser-Schichtlage (6₂) aufweist, die durch unterschiedliche, MR-unsichtbare Kunststoffmaterialien gebildet sind, und
- einem MR-Marker (7) aus MR-sichtbarem Material, der wenigstens ein MR-Markerelement aufweist, das wenigstens teilweise in den mehrlagigen Schichtaufbau der Ummantelung integriert oder von diesem umgeben ist,
- wobei die Faser-Schichtlage (6₂) durch ein Gewebe- oder Fasermaterial aus einer einlagigen, lückenlosen Anordnung von Fasern (6₂a) gebildet ist, die parallel nebeneinanderliegend oder mit einer wählbaren axialen Fasersteigung schräg verlaufend oder schraubenförmig um die darunterliegende Vollmaterial-Schichtlage (6₁) gewickelt angeordnet sind.

2. Medizinischer Führungsdraht nach Anspruch 1, weiter **dadurch gekennzeichnet, dass** der MR-Marker
- im Bereich der Ummantelung ein MR-Markerelement beinhaltet, das in eine der Ummantelungs-Schichtlagen oder zwischen zwei der Ummantelungs-Schichtlagen oder zwischen den Drahtkern und die angrenzende Ummantelungs-Schichtlage eingebettet ist, und/oder
- in einem distalen, von der Ummantelung freien Abschnitt des Führungsdrahtes ein MR-Markerelement beinhaltet, das in eine Umhüllung (4) des Drahtkerns eingebettet ist.

3. Medizinischer Führungsdraht nach Anspruch 2, weiter **dadurch gekennzeichnet, dass** das MR-Markerelement
- MR-sichtbare Partikel beinhaltet, die in eine oder mehrere der Vollmaterial-Schichtlagen eingebettet sind, und/oder
- ein oder mehrere MR-Linienelemente beinhaltet, die in die Ummantelung eingebettet sind, insbesondere als über die axiale Länge der Ummantelung durchgehendes Linienelement oder als Satz eines oder mehrerer gegenüber der Ummantelung axial kürzerer Linienelemente, die mit oder ohne Versatz in Umfangsrichtung und mit oder ohne axialen Versatz sowie mit gleicher oder unterschiedlicher Länge angeordnet sind, und/oder
- von einer der Fasern der wenigstens einen Faser-Schichtlage gebildet ist, wozu die Faser ein MR-sichtbares Material enthält, und/oder
- ein oder mehrere MR-Linienelemente beinhaltet, die entlang einer schraubenförmigen oder einer in einer Längsebene des Führungsdrahtes liegenden Linie angeordnet sind, und/oder
- ein oder mehrere MR-Linienelemente beinhaltet, die als Längenmessmarkerelemente ausgeführt sind.

4. Medizinischer Führungsdraht, insbesondere nach einem der Ansprüche 1 bis 3, der für HF-Anwendungen geeignet ist, mit
- einem Drahtkern (2) und
- einer den Drahtkern wenigstens abschnittweise und mit Berührkontakt umgebenden Ummantelung (6), die einen mehrlagigen, elektrisch isolierenden Schichtaufbau besitzt, der mindestens zwei Vollmaterial-Schichtlagen (6₁, 6₃) und wenigstens eine dazwischen angeordnete Faser-Schichtlage (6₂) aufweist, die durch unterschiedliche, elektrisch isolierende Kunststoffmaterialien gebildet sind,
- wobei die Faser-Schichtlage (6₂) durch ein Gewebe- oder Fasermaterial aus einer einlagigen, lückenlosen Anordnung von Fasern (6₂a) gebildet ist, die parallel nebeneinanderliegend oder mit einer wählbaren axialen Fasersteigung schräg verlaufend oder schraubenförmig um die darunterliegende Vollmaterial-Schichtlage (61) gewickelt angeordnet sind.

5. Medizinischer Führungsdraht nach einem der Ansprüche 1 bis 4, weiter **dadurch gekennzeichnet, dass** die Ummantelung mindestens zwei Faser-Schichtlagen (6₂, 6₄, 6₆) mit unterschiedlichen axialen Fasersteigungen und/oder Faserwickelrichtungen aufweist.

6. Medizinischer Führungsdraht nach einem der Ansprüche 1 bis 5, weiter **dadurch gekennzeichnet, dass** der Drahtkern als Drahtlitze aus mehreren seil- oder litzenbildend verbundenen Einzeldrähten (2a, 2b, 2c) gebildet ist.

7. Medizinischer Führungsdraht nach Anspruch 6, weiter **dadurch gekennzeichnet, dass** der MR-Marker ein MR-sichtbares Material (7₇) beinhaltet, das in Zwischenräumen zwischen den Einzeldrähten des als Drahtkernlitze gebildeten Drahtkerns eingebracht ist.

8. Medizinischer Führungsdraht nach einem der Ansprüche 1 bis 7, weiter **dadurch gekennzeichnet, dass** die Ummantelung den Drahtkern wenigstens in einem an einen distalen Abschnitt (3) anschließenden Schaftabschnitt (5) umgibt und mit einer höheren Biegesteifigkeit ausgeführt ist als der Drahtkern.

9. Medizinischer Führungsdraht nach einem der Ansprüche 1 bis 8, weiter **dadurch gekennzeichnet, dass** die Vollmaterial-Schichtlagen aus biegesteifen Kunststoffmaterialien gefertigt sind, insbesondere aus ABS-, PEEK-, PET-, Ultramid- und/oder Epoxidharz-Materialien.

10. Medizinischer Führungsdraht nach einem der Ansprüche 1 bis 9, weiter **dadurch gekennzeichnet, dass** die Faser-Schichtlagen aus Glasfasern, Aramidfasern und/oder Polyesterfasern gebildet sind.

11. Medizinischer Führungsdraht nach einem der Ansprüche 1 bis 10, weiter **gekennzeichnet durch** eine den Drahtkern in einem distalen Abschnitt mit Berührkontakt umgebenden Umhüllung (4) und ein MR-sichtbares, draht- oder rohrförmiges Hilfselement (11, 12) aus einem nichtmagnetischen Material, wobei das Hilfselement in einem distalen Führungsdrahtabschnitt angeordnet und in die distale Umhüllung (4) eingebettet ist.

12. Medizinischer Führungsdraht nach Anspruch 11, weiter **dadurch gekennzeichnet, dass** das Hilfselement einen Hilfselementdraht (11) umfasst, der mit axial verlaufender Hauptkomponente neben dem Drahtkern und/oder den Drahtkern schraubenlinienförmig umgebend angeordnet ist.

13. Medizinischer Führungsdraht nach Anspruch 11 oder 12, weiter **dadurch gekennzeichnet, dass** das Hilfselement mit MR-Markermaterial dotiert und/oder beschichtet ist.

## Claims

1. A medical guide wire comprising
- a wire core (2) made of an MR-invisible material,
- a sheath (6) that surrounds the wire core at least sectionally and so as to be in touching contact therewith, said sheath having a multilayer structure which comprises at least two solid material layers (6₁, 6₃) and at least one fiber layer (6₂) which are formed by different, MR-invisible plastics materials, and
- an MR marker (7) which is made of MR-visible material and comprises at least one MR marker element which is integrated at least partially into the multilayer structure of the sheath or is surrounded thereby,
- wherein the fiber layer (6₂) is formed by a fabric material or fiber material from a single-layer gapless arrangement of fibers (6₂a) arranged in a parallel manner alongside one another or obliquely extending with a selectable axial fiber pitch or in a helical manner wound around the underlying solid material layer (6₁).

2. The medical guide wire as claimed in claim 1, further **characterized in that** the MR marker
- contains an MR marker element in the region of the sheath, said MR marker element being embedded in one of the sheath layers or between two of the sheath layers or between the wire core and the adjacent sheath layer, and/or
- contains an MR marker element in a distal section, free of the sheath, of the guide wire, said MR marker element being embedded in a sheath (4) of the wire core.

3. The medical guide wire as claimed in claim 2, further **characterized in that** the MR marker element
- contains MR-visible particles which are embedded in one or more of the solid material layers, and/or
- contains one or more MR line elements which are embedded in the sheath, preferably as a line element that is continuous along the axial length of the sheath or as a set of one or more line elements that are axially shorter than the sheath and are arranged with or without an offset in the circumferential direction and with or without an axial offset and with identical or different lengths, and/or
- is formed by one of the fibers of the at least one fiber layer, to which end the fiber contains an MR-visible material, and/or
- contains one or more MR line elements which are arranged along a helical line or a line located in a longitudinal plane of the guide wire, and/or
- contains one or more MR line elements which are embodied as length measuring marker elements.

4. A medical guide wire, preferably as claimed in any one of claims 1 to 3, suited for HF appliances, comprising
- a wire core (2) and
- a sheath (6) that surrounds the wire core at least sectionally and so as to be in touching contact therewith, said sheath (6) having a multilayer, electrically insulating layer structure which comprises at least two solid material layers (6₁, 6₃) and at least one fiber layer (6₂) arranged inbetween which are formed by different, electrically insulating plastics materials,
- wherein the fiber layer (6₂) is formed by a fabric material or fiber material from a single-layer gapless arrangement of fibers (6₂a) arranged in a parallel manner alongside one another or obliquely extending with a selectable axial fiber pitch or in a helical manner wound around the underlying solid material layer (6₁).

5. The medical guide wire as claimed in any of claims 1 to 4, further **characterized in that** the sheath comprises at least two fiber layers (6₂, 6₄, 6₆) having different axial fiber pitches and/or fiber winding directions.

6. The medical guide wire as claimed in any one of claims 1 to 5, further **characterized in that** the wire core is formed as a wire strand made of a plurality of individual wires (2a, 2b, 2c) that are connected together in a cord- or strand-forming manner.

7. The medical guide wire as claimed in claim 6, further **characterized in that** the MR marker contains an MR-visible material (7₇) which is introduced into intermediate spaces between the individual wires of the wire core formed as a wire core strand.

8. The medical guide wire as claimed in any one of claims 1 to 7, further **characterized in that** the sheath surrounds the wire core at least in a shaft section (5) adjoining a distal section (3), and is embodied with greater flexural rigidity than the wire core.

9. The medical guide wire as claimed in any one of claims 1 to 8, further **characterized in that** the solid material layers are formed from flexurally rigid plastics materials, preferably from ABS, PEEK, PET, Ultramid and/or epoxy resin materials.

10. The medical guide wire as claimed in any one of claims 1 to 9, further **characterized in that** the fiber layers are formed from glass fibers, aramid fibers and/or polyester fibers.

11. The medical guide wire as claimed in any one of claims 1 to 10, further **characterized by** a sleeve (4) that surrounds the wire core with touching contact in a distal section, and an MR-visible, wire-like or tubular auxiliary element (11, 12) made of a non-magnetic material, wherein the auxiliary element is arranged in a distal guide wire section and is embedded in the distal sleeve (4) .

12. The medical guide wire as claimed in claim 11, further **characterized in that** the auxiliary element comprises an auxiliary element wire (11) which is arranged with an axially extending main component alongside the wire core and/or so as to surround the wire core in a helical manner.

13. The medical guide wire as claimed in claim 11 or 12, further **characterized in that** the auxiliary element is doped and/or coated with MR marker material.

## Revendications

1. Fil de guidage médical comportant
- un coeur de fil (2) en un matériau invisible en RM,
- une gaine (6) entourant le coeur de fil au moins partiellement et en contact direct, qui possède une structure stratifiée à plusieurs couches, qui présente au moins deux couches de matériau plein (6₁, 6₃) et au moins une couche de fibres (6₂), qui sont formées de matériaux à base de matière plastique différents, invisibles en RM, et
- un marqueur RM (7) en matériau visible en RM, qui présente au moins un élément de marqueur RM, qui est intégré au moins en partie dans la structure stratifiée à plusieurs couches de la gaine ou qui est entouré par celle-ci,
- dans lequel la couche de fibres (6₂) est formée par un matériau de tissu ou de fibres en un agencement à une seule couche ininterrompu de fibres (6₂a), qui sont disposées parallèlement les unes à côté des autres ou sont enroulées en oblique ou en hélice avec un pas axial sélectionnable des fibres autour de la couche de matériau plein sous-jacente (6₁).

2. Fil de guidage médical selon la revendication 1, **caractérisé en outre en ce que** le marqueur RM
- contient dans la région de la gaine un élément de marqueur RM, qui est incorporé dans une des couches de la gaine ou entre deux couches de la gaine ou entre le coeur de fil et la couche adjacente de la gaine, et/ou
- contient dans une partie distale du fil de guidage dépourvue de gaine, un élément de marqueur RM, qui est incorporé dans un gainage (4) du coeur de fil.

3. Fil de guidage médical selon la revendication 2, **caractérisé en outre en ce que** l'élément de marqueur RM
- contient des particules visibles en RM, qui sont incorporées dans une ou plusieurs des couches de matériau plein, et/ou
- contient un ou plusieurs élément(s) de ligne RM, qui sont incorporés dans la gaine, en particulier sous forme d'élément de ligne continu sur la longueur axiale de la gaine ou comme ensemble d'un ou de plusieurs élément(s) de ligne axialement plus courts que la gaine, qui sont disposés avec ou sans décalage en direction périphérique et avec ou sans décalage axial ainsi qu'avec une longueur identique ou différente, et/ou
- est formé par une des fibres de ladite au moins une couche de fibres, la fibre contenant à cet effet un matériau visible en RM, et/ou
- contient un ou plusieurs élément(s) de ligne RM, qui sont disposés le long d'une ligne hélicoïdale ou d'une ligne située dans un plan longitudinal du fil de guidage, et/ou
- un ou plusieurs éléments de ligne RM, qui sont réalisés sous forme d'éléments de marqueur de mesure de longueur.

4. Fil de guidage médical, en particulier selon l'une quelconque des revendications 1 à 3, qui convient pour des applications à HF, avec
- un coeur de fil (2) et
- une gaine (6) entourant le coeur de fil au moins en partie et en contact direct, qui possède une structure stratifiée électriquement isolante à plusieurs couches, qui présente au moins deux couches en matériau plein (6₁, 6₃) et au moins une couche de fibres (6₂) disposée entre celles-ci, qui sont formées par des matériaux à base de matière plastique électriquement isolants différents,
- dans lequel la couche de fibres (6₂) est formée par un matériau de tissu ou de fibres en un agencement à une seule couche ininterrompu de fibres (6₂a), qui sont disposées parallèlement les unes à côté des autres ou sont enroulées en oblique ou en hélice avec un pas axial sélectionnable des fibres autour de la couche de matériau plein sous-jacente (6₁).

5. Fil de guidage médical selon l'une quelconque des revendications 1 à 4, **caractérisé en outre en ce que** la gaine présente au moins deux couches de fibres (6₂, 6₄, 6₆) avec des pas axiaux des fibres différents et/ou des directions d'enroulement des fibres différentes.

6. Fil de guidage médical selon l'une quelconque des revendications 1 à 5, **caractérisé en outre en ce que** le coeur de fil est réalisé sous forme de toron de plusieurs fils individuels (2a, 2b, 2c) assemblés en formant un câble ou un toron.

7. Fil de guidage médical selon la revendication 6, **caractérisé en outre en ce que** le marqueur RM contient un matériau visible en RM (7₇), qui est placé dans des espaces intermédiaires entre les fils individuels du coeur de fil réalisé en forme de toron de coeur de fil.

8. Fil de guidage médical selon l'une quelconque des revendications 1 à 7, **caractérisé en outre en ce que** la gaine entoure le coeur de fil au moins dans une partie de tige (5) se raccordant à une partie distale (3) et est réalisée avec une rigidité de flexion plus élevée que le coeur de fil.

9. Fil de guidage médical selon l'une quelconque des revendications 1 à 8, **caractérisé en outre en ce que** les couches de matériau plein sont fabriquées en matériaux à base de matière plastique rigides en flexion, en particulier en matériaux à base de ABS, PEEK, PET, résine Ultramid et/ou époxy.

10. Fil de guidage médical selon l'une quelconque des revendications 1 à 9, **caractérisé en outre en ce que** les couches de fibres sont formées de fibres de verre, de fibres d'aramide et/ou de fibres de polyester.

11. Fil de guidage médical selon l'une quelconque des revendications 1 à 10, **caractérisé en outre par** un gainage (4) entourant le coeur de fil en contact direct dans une partie distale et un élément auxiliaire (11, 12) en forme de fil ou de tube visible en RM en un matériau non magnétique, dans lequel l'élément auxiliaire est disposé dans une partie distale du fil de guidage et est incorporé dans le gainage distal (4).

12. Fil de guidage médical selon la revendication 11, **caractérisé en outre en ce que** l'élément auxiliaire comprend un fil d'élément auxiliaire (11), qui est disposé avec une composante principale orientée axialement à côté du coeur de fil et/ou entourant le coeur de fil sous forme hélicoïdale.

13. Fil de guidage médical selon une revendication 11 ou 12, **caractérisé en outre en ce que** l'élément auxiliaire est dopé et/ou revêtu avec un matériau de marqueur RM.
